# EUROPEAN PATENT APPLICATION

(11) **EP 4 365 200 A1**
(43) Date of publication of application: **08.05.2024**
(21) Application number: 22832015.6
(22) Date of filing: 28.06.2022
(51) Int. Cl.: C07K 16/28, A61K 39/395

(54) **ANTI-CD40 ANTIBODY, ANTIGEN-BINDING FRAGMENT AND MEDICAL USE THEREOF**

(30) Priority: 28.06.2021 CN 202110722124
(71) Applicant: Jiangsu Hengrui Pharmaceuticals Co., Ltd., Lianyungang, Jiangsu 222047 (CN); Shanghai Shengdi Pharmaceutical Co., Ltd, Shanghai 201210 (CN)
(72) Inventor: LIN, Yuan, Shanghai 201210 (CN); SU, Lu, Shanghai 201210 (CN); LIN, Kan, Shanghai 201210 (CN); LIAO, Cheng, Shanghai 201210 (CN)
(74) Representative: Potter Clarkson
(86) International application number: PCT/CN2022/101780
(87) International publication number: WO 2023/274201

(57) **Abstract**

Provided are an anti-CD40 antibody, an antigen-binding fragment and a medical use thereof, as well as a pharmaceutical composition containing the anti-CD40 antibody or antigen-binding fragment thereof, and a method for treating and preventing disease, in particular a method for treating autoimmune disease.

## Description

The present application claims priority to Chinese Patent Application No. 202110722124.0 filed on June 28, 2021.

### TECHNICAL FIELD

The present disclosure relates to the field of biopharmaceutics, and particularly to the field of treating or interfering with diseases associated with the CD40/CD40L signaling pathway. Specifically, the present disclosure relates to a CD40 antibody, an antigen-binding fragment thereof and a pharmaceutical composition thereof, as well as a method for treating an autoimmune disease and related pharmaceutical use.

### BACKGROUND

CD40, which belongs to the tumor necrosis factor receptor (TNFR) superfamily, is a type I transmembrane glycoprotein localized on the cell membrane surface, has a molecular weight of about 48 kDa, and plays an important role in the immune system. CD40 is expressed in a variety of immune cells, such as B cells, dendritic cells, monocytes and macrophages, as well as on platelets, and under certain conditions can be expressed on eosinophils and parenchymal cells. The natural ligand for CD40 is CD154 or CD40L, a type II transmembrane protein, the expression of which can be induced on a variety of cell types, including activated CD4+ T cells, NK cells, platelets and B cells (Pucino V et al., 2020).

CD40L, upon binding to CD40, recruits TRAFs and mediates downstream signaling through the NF-kB, JNK and MAPK pathways, producing a variety of cell type-dependent activation outcomes, including immune cell activation and proliferation, inflammatory factor and chemokine secretion, and the like. (Vonderheide RH et al., 2007). For example, signaling through these pathways is necessary for several important effector functions of the adaptive immune system, including primary T cell-dependent antibody responses (TDARs), B cell proliferation, germinal center (GC) formation, immunoglobulin (Ig) isotype switching, somatic mutation, and differentiation of memory B cells and plasma cells (Foy TM et al., 1993; Foy TM et al., 1994). In addition to the effects on B cells, CD40 pathway activation provides important signals for DC maturation and function, as well as the survival of monocytes and macrophages and cytokine secretion (Caux, C et al., 1994).

Dysregulation of the CD40 signaling pathway can lead to autoimmune diseases (Kamell JL et al., 2018). The CD40-CD40L signaling pathway has been found to be involved in the function of parenchymal cells in inflamed tissues: activated epithelial cells from sites such as the kidney, salivary glands and skin that can secrete chemokines are able to respond to CD40. Moreover, the expression levels of CD40 or CD40L are elevated in the lesion sites of patients with atherosclerosis and in preclinical models of atherosclerosis. CD40 can stimulate and induce the expression of matrix-degrading enzymes, promoting the expression of tissue factors in cell types related to the pathogenesis of atherosclerosis, such as endothelial cells, smooth muscle cells and macrophages (Michel NA et al., 2017). The CD40 pathway up-regulates the production of inflammatory factors such as IL-1, IL-6 and IL-8, as well as adhesion molecules including intercellular adhesion molecule-1 (ICAM-1), E-selectin and vascular cell adhesion molecule (VCAM). The CD40/CD40L interaction has also been used to prevent graft rejection. In a study of renal allografts in cynomolgus monkeys, the use of chimeric anti-CD40 antagonist ch5D12 demonstrated that CD40 antagonism was sufficient to improve the condition and extend the average survival time to over 100 days. When ch5D12 was combined with an anti-CD86 antibody and given only at the start of the allograft study, followed by extended treatment with cyclosporine, an average survival time of over 4 years was achieved, suggesting that such a combination can potentially induce immune tolerance (Haanstra et al., 2005). Numerous preclinical studies provide evidence for the key role of the CD40/CD40L interaction in promoting T cell-dependent immune responses. Therefore, blocking CD40 signaling is considered a suitable and desirable therapeutic strategy for suppressing pathogenic autoimmune responses in diseases such as rheumatoid arthritis, systemic lupus erythematosus and Sjögren's syndrome. Currently, no anti-CD40 antibodies have been approved as treatments for such diseases. Thus, there is still an urgent need in the art for therapeutic agents that can intervene in the CD40-CD40L interaction and block CD40 signaling. The present disclosure provides therapeutic humanized anti-CD40 antibodies that specifically bind to CD40 and possess excellent antigen-binding specificity, affinity, and pharmacokinetic and pharmacodynamic properties useful for intervening in or treating diseases related to the CD40 signaling pathway, particularly autoimmune diseases. Additionally, combinations of the therapeutic humanized anti-CD40 antibodies with tacrolimus are provided for treating graft-versus-host disease or alleviating graft rejection.

### SUMMARY

The present disclosure provides an anti-CD40 antibody and an antigen-binding fragment thereof, a coding polynucleotide thereof, a vector comprising the polynucleotide, a host cell, a pharmaceutical composition comprising the antibody or the antigen-binding fragment thereof, a method for using same to treat or intervene in autoimmune diseases (including graft-versus-host disease and graft rejection), and related pharmaceutical use.

### Anti-CD40 Antibody or Antigen-Binding Fragment Thereof

In one aspect, in some embodiments, the present disclosure provides an anti-CD40 antibody and an antigen-binding fragment thereof, comprising:
a heavy chain HCDR1, comprising the sequence set forth in SEQ ID NO: 23;
a heavy chain HCDR2, comprising the sequence set forth in SEQ ID NO: 24;
a heavy chain HCDR3, comprising the sequence set forth in SEQ ID NO: 25;
a light chain LCDR1, comprising the sequence QX₁SEDISSNLX₂ (SEQ ID NO: 74), wherein X₁ is selected from the group consisting of A and S, and X₂ is selected from the group consisting of A and S;
a light chain LCDR2, comprising the sequence X₃ASNLAS (SEQ ID NO: 75), wherein X₃ is selected from the group consisting of A and P; and
a light chain LCDR3, comprising the sequence QGX₄YWX₅X₆X₇SX₈FGX₉X₁₀ (SEQ ID NO: 76), wherein X₄ is selected from the group consisting of A and G, X₅ is selected from the group consisting of S and T, X₆ is selected from the group consisting of S and G, X₇ is selected from the group consisting of T and S, X₈ is selected from the group consisting of N and Y, X₉ is selected from the group consisting of N, S, T and Q, and X₁₀ is selected from the group consisting of V and G.

In some specific embodiments, provided is an anti-CD40 antibody or an antigen-binding fragment thereof, comprising:
a heavy chain HCDR1, a heavy chain HCDR2 and a heavy chain HCDR3, comprising the sequences set forth in SEQ ID NOs: 23, 24 and 25, respectively; and/or a light chain LCDR1, a light chain LCDR2 and a light chain LCDR3, comprising the sequences set forth in SEQ ID NOs: 26, 27 and 28, respectively;
a heavy chain HCDR1, a heavy chain HCDR2 and a heavy chain HCDR3, comprising the sequences set forth in SEQ ID NOs: 23, 24 and 25, respectively; and/or a light chain LCDR1, comprising the sequence set forth in SEQ ID NO: 29; a light chain LCDR2, comprising the sequence set forth in SEQ ID NO: 30; and a light chain LCDR3, comprising the sequence QGGYWTSTSNFGX₉X₁₀ (SEQ ID NO: 73), wherein X₉ is selected from the group consisting of N, S, T and Q, and X₁₀ is selected from the group consisting of V and G; or
a heavy chain HCDR1, a heavy chain HCDR2 and a heavy chain HCDR3, comprising the sequences set forth in SEQ ID NOs: 23, 24 and 25, respectively; and/or a light chain LCDR1, a light chain LCDR2 and a light chain LCDR3, comprising the sequences set forth in SEQ ID NOs: 29, 27 and 32, respectively.

In some specific embodiments, provided is an anti-CD40 antibody or an antigen-binding fragment thereof, comprising:
a heavy chain HCDR1, a heavy chain HCDR2 and a heavy chain HCDR3, comprising the sequences set forth in SEQ ID NOs: 23, 24 and 25, respectively; a light chain LCDR1, comprising the sequence set forth in SEQ ID NO: 29; a light chain LCDR2, comprising the sequence set forth in SEQ ID NO: 30; and a light chain LCDR3, comprising the sequence set forth in any one of SEQ ID NOs: 69-72.

In some specific embodiments, the present disclosure provides an anti-CD40 antibody or an antigen-binding fragment thereof, comprising any one of the aforementioned HCDR1, HCDR2, HCDR3, LCDR1, LCDR2 and LCDR3, or any combination thereof.

In another aspect, in some embodiments, the present disclosure provides an anti-CD40 antibody or an antigen-binding fragment thereof, comprising:
a heavy chain HCDR1, comprising the sequence SYGVX₁₁ (SEQ ID NO: 88), wherein X₁₁ is selected from the group consisting of S and T;
a heavy chain HCDR2, comprising the sequence X₁₂IX₁₃SX₁₄GX₁₅X₁₆YYAX₁₇WAX₁₈S (SEQ ID NO: 89), wherein X₁₂ is selected from the group consisting of A and G, X₁₃ is selected from the group consisting of G and A, X₁₄ is selected from the group consisting of T, S and D, X₁₅ is selected from the group consisting of T and S, X₁₆ is selected from the group consisting of T and A, X₁₇ is selected from the group consisting of S and N, and X₁₈ is selected from the group consisting of K and R;
a heavy chain HCDR3, comprising the sequence GGITX₁₉YAX₂₀ (SEQ ID NO: 90), wherein X₁₉ is selected from the group consisting of A and V, and X₂₀ is selected from the group consisting of I and M;
a light chain LCDR1, comprising the sequence QASX₂₁X₂₂IX₂₃X₂₄X₂₅LA (SEQ ID NO: 91), wherein X₂₁ is selected from the group consisting of Q and E, X₂₂ is selected from the group consisting of S and D, X₂₃ is selected from the group consisting of S and T, X₂₄ is selected from the group consisting of N, Q, S and T, and X₂₅ is selected from the group consisting of V and G;
a light chain LCDR2, comprising the sequence set forth in SEQ ID NO: 37; and
a light chain LCDR3, comprising the sequence QSYX₂₆X₂₇SX₂₈X₂₉TX₃₀ (SEQ ID NO: 92), wherein X₂₆ is selected from the group consisting of F and Y, X₂₇ is selected from the group consisting of S, D and N, X₂₈ is selected from the group consisting of S and F, X₂₉ is selected from the group consisting of S, T and Y, and X₃₀ is selected from the group consisting of V and I.

In some specific embodiments, provided is an anti-CD40 antibody or an antigen-binding fragment thereof, comprising:
a heavy chain HCDR1, a heavy chain HCDR2 and a heavy chain HCDR3, comprising the sequences set forth in SEQ ID NOs: 33, 34 and 35, respectively; and/or a light chain LCDR1, a light chain LCDR2 and a light chain LCDR3, comprising the sequences set forth in SEQ ID NOs: 36, 37 and 38, respectively;
a heavy chain HCDR1, a heavy chain HCDR2 and a heavy chain HCDR3, comprising the sequences set forth in SEQ ID NOs: 39, 40 and 41, respectively; and/or a light chain LCDR1, a light chain LCDR2 and a light chain LCDR3, comprising the sequences set forth in SEQ ID NOs: 42, 37 and 43, respectively;
a heavy chain HCDR1, a heavy chain HCDR2 and a heavy chain HCDR3, comprising the sequences set forth in SEQ ID NOs: 39, 44 and 35, respectively; and/or a light chain LCDR1, a light chain LCDR2 and a light chain LCDR3, comprising the sequences set forth in SEQ ID NOs: 45, 37 and 46, respectively;
a heavy chain HCDR1, a heavy chain HCDR2 and a heavy chain HCDR3, comprising the sequences set forth in SEQ ID NOs: 39, 47 and 41, respectively; and/or a light chain LCDR1, a light chain LCDR2 and a light chain LCDR3, comprising the sequences set forth in SEQ ID NOs: 36, 37 and 48, respectively; or
a heavy chain HCDR1, a heavy chain HCDR2 and a heavy chain HCDR3, comprising the sequences set forth in SEQ ID NOs: 39, 47 and 49, respectively; and/or a light chain LCDR1, comprising the sequence QASQSISX₂₄X₂₅LA (SEQ ID NO: 87), wherein X₂₄ is selected from the group consisting of N, Q, S and T, and X₂₅ is selected from the group consisting of V and G; a light chain LCDR2, comprising the sequence set forth in SEQ ID NO: 50; and a light chain LCDR3, comprising the sequence set forth in SEQ ID NO: 48.

In some specific embodiments, provided is an anti-CD40 antibody or an antigen-binding fragment thereof, comprising:
a heavy chain HCDR1, a heavy chain HCDR2 and a heavy chain HCDR3, comprising the sequences set forth in SEQ ID NOs: 39, 47 and 49, respectively; a light chain LCDR1, comprising the sequence set forth in any one of SEQ ID NOs: 83-86; a light chain LCDR2 and a light chain LCDR3, comprising the sequences set forth in SEQ ID NOs: 50 and 48, respectively.

In some specific embodiments, the present disclosure provides an anti-CD40 antibody or an antigen-binding fragment thereof, comprising any one of the aforementioned HCDR1, HCDR2, HCDR3, LCDR1, LCDR2 and LCDR3, or any combination thereof.

In another aspect, in some embodiments, the present disclosure provides an anti-CD40 antibody or an antigen-binding fragment thereof, comprising: a heavy chain HCDR1, a heavy chain HCDR2 and a heavy chain HCDR3, comprising the sequences set forth in SEQ ID NOs: 51, 52 and 53, respectively; and/or a light chain LCDR1, a light chain LCDR2 and a light chain LCDR3, comprising the sequences set forth in SEQ ID NOs: 54, 55 and 56, respectively.

In some embodiments, the present disclosure provides an anti-CD40 antibody or an antigen-binding fragment thereof, comprising: a heavy chain HCDR1, a heavy chain HCDR2 and a heavy chain HCDR3, comprising the sequences set forth in SEQ ID NOs: 57, 58 and 59, respectively; and/or a light chain LCDR1, a light chain LCDR2 and a light chain LCDR3, comprising the sequences set forth in SEQ ID NOs: 60, 61 and 62, respectively.

In some specific embodiments, the present disclosure provides an anti-CD40 antibody or an antigen-binding fragment thereof, comprising any one of the aforementioned HCDR1, HCDR2, HCDR3, LCDR1, LCDR2 and LCDR3, or any combination thereof.

In another aspect, the present disclosure provides an anti-CD40 antibody or an antigen-binding fragment thereof, comprising a heavy chain variable region (VH) and a light chain variable region (VL), wherein:
a-1) the VH comprises a HCDR1, a HCDR2 and a HCDR3 in a VH set forth in SEQ ID NO: 1, and the VL comprises a LCDR1, a LCDR2 and a LCDR3 in a VL set forth in SEQ ID NO: 2;
a-2) the VH comprises a HCDR1, a HCDR2 and a HCDR3 in a VH set forth in SEQ ID NO: 3, and the VL comprises a LCDR1, a LCDR2 and a LCDR3 in a VL set forth in SEQ ID NO: 4;
a-3) the VH comprises a HCDR1, a HCDR2 and a HCDR3 in a VH set forth in SEQ ID NO: 5, and the VL comprises a LCDR1, a LCDR2 and a LCDR3 in a VL set forth in SEQ ID NO: 6;
a-4) the VH comprises a HCDR1, a HCDR2 and a HCDR3 in a VH set forth in any one of SEQ ID NOs: 67 and 68, and the VL comprises a LCDR1, a LCDR2 and a LCDR3 in a VL set forth in any one of SEQ ID NOs: 63-66;
b-1) the VH comprises a HCDR1, a HCDR2 and a HCDR3 in a VH set forth in SEQ ID NO: 7, and the VL comprises a LCDR1, a LCDR2 and a LCDR3 in a VL set forth in SEQ ID NO: 8;
b-2) the VH comprises a HCDR1, a HCDR2 and a HCDR3 in a VH set forth in SEQ ID NO: 9, and the VL comprises a LCDR1, a LCDR2 and a LCDR3 in a VL set forth in SEQ ID NO: 10;
b-3) the VH comprises a HCDR1, a HCDR2 and a HCDR3 in a VH set forth in SEQ ID NO: 11, and the VL comprises a LCDR1, a LCDR2 and a LCDR3 in a VL set forth in SEQ ID NO: 12;
b-4) the VH comprises a HCDR1, a HCDR2 and a HCDR3 in a VH set forth in SEQ ID NO: 13, and the VL comprises a LCDR1, a LCDR2 and a LCDR3 in a VL set forth in SEQ ID NO: 14;
b-5) the VH comprises a HCDR1, a HCDR2 and a HCDR3 in a VH set forth in SEQ ID NO: 15, and the VL comprises a LCDR1, a LCDR2 and a LCDR3 in a VL set forth in SEQ ID NO: 16;
b-6) the VH comprises a HCDR1, a HCDR2 and a HCDR3 in a VH set forth in SEQ ID NO: 17, and the VL comprises a LCDR1, a LCDR2 and a LCDR3 in a VL set forth in SEQ ID NO: 18;
b-7) the VH comprises a HCDR1, a HCDR2 and a HCDR3 in a VH set forth in any one of SEQ ID NOs: 81 and 82, and the VL comprises a LCDR1, a LCDR2 and a LCDR3 in a VL set forth in any one of SEQ ID NOs: 77-80;
c) the VH comprises a HCDR1, a HCDR2 and a HCDR3 in a VH set forth in SEQ ID NO: 19, and the VL comprises a LCDR1, a LCDR2 and a LCDR3 in a VL set forth in SEQ ID NO: 20; or
d) the VH comprises a HCDR1, a HCDR2 and a HCDR3 in a VH set forth in SEQ ID NO: 21, and the VL comprises a LCDR1, a LCDR2 and a LCDR3 in a VL set forth in SEQ ID NO: 22;
wherein the CDRs are defined according to the Kabat, IMGT, Chothia, AbM or Contact numbering scheme. In some specific embodiments, the CDRs are defined according to the Kabat numbering scheme.

In some embodiments, the aforementioned anti-CD40 antibody or antigen-binding fragment thereof is a recombinant antibody.

In some embodiments, the aforementioned anti-CD40 antibody or antigen-binding fragment thereof is a rabbit antibody, a chimeric antibody, a humanized antibody, a human antibody or an antigen-binding fragment thereof.

In some embodiments, when the aforementioned anti-CD40 antibody or antigen-binding fragment thereof is a humanized antibody, heavy chain framework regions are derived from IGHV2-26*01, IGHV4-30-4*02, IGHV4-4*08 and IGHJ1*01, and/or light chain framework regions are derived from IGkV1-13*02, IGkV1-9*01, IGkV1-6*01 and IGKJ4*01. For example, heavy chain framework regions FR1-FR3 are derived from IGHV2-26*01, IGHV4-30-4*02 and IGHV4-4*08, and a heavy chain framework region FR4 is derived from IGHJ1*01; light chain framework regions FR1-FR3 are derived from IGkV1-13*02, IGkV1-9*01 and IGkV1-6*01, and a light chain framework region FR4 is derived from IGKJ4*01.

In some embodiments, the aforementioned anti-CD40 antibody or antigen-binding fragment thereof comprises a VH and a VL, wherein
A-1) the amino acid sequence of the VH is set forth in SEQ ID NO: 1 or has at least 90% identity thereto, and the amino acid sequence of the VL is set forth in SEQ ID NO: 2 or has at least 90% identity thereto;
A-2) the amino acid sequence of the VH is set forth in SEQ ID NO: 3 or has at least 90% identity thereto, and the amino acid sequence of the VL is set forth in SEQ ID NO: 4 or has at least 90% identity thereto;
A-3) the amino acid sequence of the VH is set forth in SEQ ID NO: 5 or has at least 90% identity thereto, and the amino acid sequence of the VL is set forth in SEQ ID NO: 6 or has at least 90% identity thereto;
A-4) the amino acid sequence of the VH is set forth in SEQ ID NO: 67 or has at least 90% identity thereto, and the amino acid sequence of the VL is set forth in any one of SEQ ID NOs: 63-66 or has at least 90% identity thereto;
A-5) the amino acid sequence of the VH is set forth in SEQ ID NO: 68 or has at least 90% identity thereto, and the amino acid sequence of the VL is set forth in any one of SEQ ID NOs: 63-66 or has at least 90% identity thereto;
B-1) the amino acid sequence of the VH is set forth in SEQ ID NO: 7 or has at least 90% identity thereto, and the amino acid sequence of the VL is set forth in SEQ ID NO: 8 or has at least 90% identity thereto;
B-2) the amino acid sequence of the VH is set forth in SEQ ID NO: 9 or has at least 90% identity thereto, and the amino acid sequence of the VL is set forth in SEQ ID NO: 10 or has at least 90% identity thereto;
B-3) the amino acid sequence of the VH is set forth in SEQ ID NO: 11 or has at least 90% identity thereto, and the amino acid sequence of the VL is set forth in SEQ ID NO: 12 or has at least 90% identity thereto;
B-4) the amino acid sequence of the VH is set forth in SEQ ID NO: 13 or has at least 90% identity thereto, and the amino acid sequence of the VL is set forth in SEQ ID NO: 14 or has at least 90% identity thereto;
B-5) the amino acid sequence of the VH is set forth in SEQ ID NO: 15 or has at least 90% identity thereto, and the amino acid sequence of the VL is set forth in SEQ ID NO: 16 or has at least 90% identity thereto;
B-6) the amino acid sequence of the VH is set forth in SEQ ID NO: 17 or has at least 90% identity thereto, and the amino acid sequence of the VL is set forth in SEQ ID NO: 18 or has at least 90% identity thereto;
B-7) the amino acid sequence of the VH is set forth in SEQ ID NO: 81 or has at least 90% identity thereto, and the amino acid sequence of the VL is set forth in any one of SEQ ID NOs: 77-80 or has at least 90% identity thereto;
B-8) the amino acid sequence of the VH is set forth in SEQ ID NO: 82 or has at least 90% identity thereto, and the amino acid sequence of the VL is set forth in any one of SEQ ID NOs: 77-80 or has at least 90% identity thereto;
C) the amino acid sequence of the VH is set forth in SEQ ID NO: 19 or has at least 90% identity thereto, and the amino acid sequence of the VL is set forth in SEQ ID NO: 20 or has at least 90% identity thereto; or
D) the amino acid sequence of the VH is set forth in SEQ ID NO: 21 or has at least 90% identity thereto, and the amino acid sequence of the VL is set forth in SEQ ID NO: 22 or has at least 90% identity thereto.

In some embodiments, the aforementioned anti-CD40 antibody or antigen-binding fragment thereof is an IgG antibody or an antigen-binding fragment thereof, e.g., an IgG1, IgG2, IgG2 or IgG4 antibody or an antigen-binding fragment thereof, e.g., an IgG1 antibody having the mutation N297A or an antigen-binding fragment thereof, e.g., an IgG1 antibody having one of L234A, L235A, M252Y, S254T and T256E, or any combination thereof, or an antigen-binding fragment thereof.

In some embodiments, the aforementioned antigen-binding fragment of the anti-CD40 antibody is a Fab, an Fv, an sFv, a Fab', a F(ab')₂, a linear antibody, a single-chain antibody, an scFv, an sdAb, an sdFv, a nanobody, a peptibody, a domain antibody, and a multispecific antibody (bispecific antibody, diabody, triabody and tetrabody, tandem di-scFv, tandem tri-scFv), for example, an scFv, Fv, Fab or Fab' fragment.

In some embodiments, of the aforementioned antigen-binding fragment of the CD40 antibody, the full-length amino acid sequence of a heavy chain is set forth in SEQ ID NO: 93 or 97 or has at least 90% identity thereto, and the full-length amino acid sequence of a light chain is set forth in SEQ ID NO: 94 or has at least 90% identity thereto; or
the full-length amino acid sequence of a heavy chain is set forth in SEQ ID NO: 95 or 98 or has at least 90% identity thereto, and the full-length amino acid sequence of a light chain is set forth in SEQ ID NO: 96 or has at least 90% identity thereto.

As described above, "at least 90% identity" includes, for example, at least 91%, at least 92%, at least 93%, at least 94%, at least 95%, at least 96%, at least 97%, at least 98% and at least 99% identity.

In some embodiments, of the aforementioned anti-CD40 antibody or antigen-binding fragment thereof, the heavy chain variable region has 0 to 10 (1, 2, 3, 4, 5, 6, 7, 8, 9 or 10) amino acid alterations, and the light chain variable region has 0 to 10 (1, 2, 3, 4, 5, 6, 7, 8, 9 or 10) amino acid alterations. In some specific embodiments, the amino acid alterations are conservative replacements, substitutions or modifications, and/or deletions or additions that do not affect function.

In some embodiments, provided is an anti-CD40 antibody or an antigen-binding fragment, which binds to or competes for binding to the same epitope with the aforementioned anti-CD40 antibody or antigen-binding fragment thereof.

In some embodiments, provided is an anti-CD40 antibody or an antigen-binding fragment, which blocks the binding of the aforementioned anti-CD40 antibody or antigen-binding fragment thereof to CD40 (e.g., human CD40).

In some embodiments, provided is an anti-CD40 antibody or an antigen-binding fragment, the binding of which to CD40 (e.g., human CD40) is blocked by the aforementioned anti-CD40 antibody or antigen-binding fragment thereof.

In some embodiments, the aforementioned anti-CD40 antibody or antigen-binding fragment has at least one of the following:
(i) binding to human CD40 with a K_{D} of 10 nM or lower; and
(ii) having no significant agonistic activity.

In some embodiments, the aforementioned anti-CD40 antibody or antigen-binding fragment reduces the binding of a CD40 ligand to CD40 by at least 45%, at least 50%, at least 60%, at least 75%, at least 80%, at least 90% or at least 95%.

In some embodiments, the aforementioned anti-CD40 antibody or antigen-binding fragment binds to human CD40 with a K_{D} of 10⁻⁷ M, 10⁻¹ M, 10⁻⁹ M, 10⁻¹⁰ M, 10⁻¹¹ M or lower.

In some embodiments, provided is a CD40-binding molecule, comprising any of the aforementioned anti-CD40 antibodies or antigen-binding fragments thereof.

In some embodiments, provided is a conjugate, comprising the aforementioned anti-CD40 antibody or antigen-binding fragment thereof. For example, the conjugate is an antibody-drug conjugate.

### Polynucleotide and Vector

The present disclosure provides an isolated polynucleotide encoding the anti-CD40 antibody or the antigen-binding fragment thereof of the present disclosure. The isolated polynucleotide may be an RNA, a DNA or a cDNA. According to some embodiments of the present disclosure, the polynucleotide of the present disclosure is an isolated polynucleotide.

The present disclosure also provides a DNA molecule encoding any of the aforementioned anti-CD40 antibodies or antigen-binding fragments thereof of the present disclosure.

The polynucleotide of the present disclosure may be in the form of, may be present in, and/or may be part of a vector, such as a plasmid, cosmid, YAC, or viral vector. The vector may especially be an expression vector, i.e., a vector that can provide for the expression of a VEGF-binding molecule or a conjugate thereof *in vitro* and/or *in vivo* (i.e., in a suitable host cell, host organism and/or expression system). The expression vector generally comprises at least one of the polynucleotides of the present disclosure, which is operably linked to one or more suitable expression regulatory elements (e.g., promoters, enhancers, terminators, and the like). The selection of the elements and their sequences for expression in a particular host is within the knowledge of those skilled in the art. Regulatory elements and other elements useful or necessary for expressing the anti-CD40 antibody or the antigen-binding fragment thereof of the present disclosure are, for example, promoters, enhancers, terminators, integration factors, selection markers, leader sequences or reporter genes.

The polynucleotide of the present disclosure may be prepared or obtained by known means (e.g., by automatic DNA synthesis and/or recombinant DNA techniques) based on information on the amino acid sequence of the polypeptide of the present disclosure, and/or may be isolated from a suitable natural source.

### Host Cell

The present disclosure provides a recombinant host cell that expresses the anti-CD40 antibody or the antigen-binding fragment thereof or the conjugate of the present disclosure or comprises the polynucleotide or vector of the present disclosure. In some embodiments, the host cell is a bacterial cell, a fungal cell or a mammalian cell. Bacterial cells include, for example, cells of gram-negative bacterial strains (e.g., *Escherichia coli* strains, *Proteus* strains, and *Pseudomonas* strains), and gram-positive bacterial strains (e.g., *Bacillus* strains, *Streptomyces* strains, *Staphylococcus* strains, and *Lactococcus* strains).

Fungal cells include, for example, cells of species of *Trichoderma*, *Neurospora*, and *Aspergillus*; or cells of species of *Saccharomyces* (e.g., *Saccharomyces cerevisiae*), *Schizosaccharomyces* (e.g., *Schizosaccharomyces pombe*), *Pichia* (*Pichia pastoris* and *Pichia methanolica*), and *Hansenula.*

Mammalian cells include, for example, HEK293 cells, CHO cells, BHK cells, HeLa cells, COS cells, and the like.

However, amphibian cells, insect cells, plant cells, and any other cells used in the art for expressing heterologous proteins may also be used in the present disclosure.

In one embodiment, the host cell used in the present disclosure is unable to develop into a complete plant or animal individual.

### Preparation Method

The present disclosure also provides a method for preparing the anti-CD40 antibody or the antigen-binding fragment thereof of the present disclosure, comprising:
- culturing the host cell of the present disclosure under conditions that allow the expression of the anti-CD40 antibody or the antigen-binding fragment thereof of the present disclosure; and
- collecting from the culture the anti-CD40 antibody or the antigen-binding fragment thereof expressed by the host cell; and
- optionally, further purifying and/or modifying the anti-CD40 antibody or the antigen-binding fragment thereof of the present disclosure.

The present disclosure provides a method for preparing a conjugate, comprising conjugating or modifying a drug onto the anti-CD40 antibody or the antigen-binding fragment thereof of the present disclosure.

The anti-CD40 antibody or the antigen-binding fragment thereof of the present disclosure can be produced intracellularly (e.g., in the cytoplasm, in the periplasm, or in inclusion bodies) in a cell as described above, followed by isolation from the host cell and optionally further purification; or it may be produced extracellularly (e.g., in the medium in which the host cell is cultured), followed by isolation from the medium and optionally further purification. For example, purification is performed using an A or G Sepharose FF column containing an adjusted buffer to wash off non-specifically bound components, and then bound antibodies are eluted by a pH gradient method, detected by SDS-PAGE, and collected. Optionally, the antibody solution can be filtered and concentrated by conventional methods. Soluble mixtures and polymers can also be removed by conventional methods, such as molecular sieves and ion exchange. The resulting product needs to be immediately frozen, e.g., at -70 °C, or lyophilized.

Methods and reagents for recombinant production of polypeptides, e.g., specific suitable expression vectors, transformation or transfection methods, selection labels, methods for inducing protein expression, culture conditions, and the like, are known in the art. Similarly, protein isolation and purification techniques suitable for use in the method for manufacturing the anti-CD40 antibody or the antigen-binding fragment thereof or the conjugate of the present disclosure are well known to those of skill in the art.

### Composition

The present disclosure provides a composition comprising the aforementioned anti-CD40 antibody or antigen-binding fragment thereof. For example, provided is a pharmaceutical composition, comprising a therapeutically or alleviation effective amount of an anti-CD40 antibody or an antigen-binding fragment thereof as described above, and at least one pharmaceutically acceptable excipient, diluent or carrier.

In some specific embodiments, a unit dose of the pharmaceutical composition may comprise 0.01 to 99 wt% of the anti-CD40 antibody or the antigen-binding fragment thereof, or a unit dose of the pharmaceutical composition comprises 0.1-2000 mg, and in some specific embodiments, 1-1000 mg, of the anti-CD40 antibody or the antigen-binding fragment thereof.

In some embodiments, provided is a combination or composition of the aforementioned anti-CD40 antibody or antigen-binding fragment thereof and one or more additional immunosuppressive agents. The composition is, for example, a pharmaceutical composition. Optionally, the pharmaceutical composition may further comprise a pharmaceutically acceptable excipient, diluent or carrier.

In one embodiment of the present disclosure, provided is a pharmaceutical composition, comprising the aforementioned anti-CD40 antibody or antigen-binding fragment thereof, tacrolimus, and a pharmaceutically acceptable excipient, diluent or carrier.

In some embodiments, provided is an article of manufacture, comprising the aforementioned anti-CD40 antibody or antigen-binding fragment thereof. Optionally, the article of manufacture comprises a container and a label. Examples of containers are bottles, syringes and test tubes. The container accommodates a composition effective in treating a condition. The label on or associated with the container indicates that the composition is used for treating the condition of choice. The composition comprises the aforementioned anti-CD40 antibody or antigen-binding fragment thereof. The article of manufacture may further comprise a second container accommodating tacrolimus, which is effective in treating the condition.

In some embodiments, provided is a product, comprising the aforementioned anti-CD40 antibody or antigen-binding fragment thereof and tacrolimus.

### Treatment Method and Pharmaceutical Use

The present disclosure provides a method for using the aforementioned anti-CD40 antibody or antigen-binding fragment thereof to treat, intervene in, prevent or diagnose a disease or condition.

Specifically, in some embodiments, the present disclosure provides use of the anti-CD40 antibody or the antigen-binding fragment thereof according to the present disclosure in the preparation of a medicament for treating or alleviating an autoimmune disease or graft-versus-host disease, or alleviating graft rejection.

Further, in some embodiments, the present disclosure provides use of the anti-CD40 antibody or the antigen-binding fragment thereof according to the present disclosure in combination with one or more additional immunosuppressive agents in the preparation of a medicament for treating or alleviating an autoimmune disease or graft-versus-host disease, or alleviating graft rejection.

In an embodiment of the present disclosure, the anti-CD40 antibody or the antigen-binding fragment thereof according to the present disclosure may be administered separately, sequentially or concurrently with one or more additional immunosuppressive agents.

In an embodiment of the present disclosure, the anti-CD40 antibody or the antigen-binding fragment thereof according to the present disclosure may be administered before, after or concurrently with one or more additional immunosuppressive agents.

Specifically, the present disclosure provides use of the anti-CD40 antibody or the antigen-binding fragment thereof according to the present disclosure in combination with tacrolimus in the preparation of a medicament for treating or alleviating an autoimmune disease or graft-versus-host disease, or alleviating graft rejection. In some embodiments, provided are a method for relieving or treating graft-versus-host disease and organ graft rejection, and related pharmaceutical use, the method comprising administering to a subject a relief or therapeutically effective amount of the aforementioned anti-CD40 antibody or antigen-binding fragment thereof or pharmaceutical composition thereof.

In some embodiments, provided are a method for relieving or treating autoimmune diseases and inflammatory diseases, and related pharmaceutical use, the method comprising administering to a subject a relief or therapeutically effective amount of the aforementioned anti-CD40 antibody or antigen-binding fragment thereof or pharmaceutical composition thereof.

In some embodiments, provided are a method for treating CD40-related disorders and diseases, and related pharmaceutical use; in some embodiments, provided are a method for inhibiting the growth or differentiation of cells of CD40-related disorders, and related pharmaceutical use; in some embodiments, provided are a method for inhibiting the growth and/or differentiation of cells that express the antigen human CD40, and related pharmaceutical use; in some embodiments, provided are a method for producing an antibody that inhibits B cells in a subject, and related pharmaceutical use; in some embodiments, provided are a method for treating immune disorders and diseases, and related pharmaceutical use. The above methods all comprise administering to a subject or cell a therapeutically or inhibitory effective amount of the aforementioned anti-CD40 antibody or antigen-binding fragment thereof or pharmaceutical composition thereof.

In some embodiments, provided are a method for inducing peripheral B cell depletion, and related pharmaceutical use, the method comprising administering to a subject an induction effective amount of the aforementioned anti-CD40 antibody or antigen-binding fragment thereof or pharmaceutical composition thereof.

In some embodiments, provided are a method for treating or alleviating a disease or condition, and related pharmaceutical use, the method comprising administering to a subject in need thereof an effective amount of the aforementioned anti-CD40 antibody or antigen-binding fragment thereof, wherein the disease or condition may or may not be related to CD40, and includes: rheumatoid arthritis, systemic lupus erythematosus, lupus nephritis, autoimmune demyelinating diseases (e.g., multiple sclerosis and allergic encephalomyelitis), endocrine opthalmopathy, uveoretinitis, systemic lupus erythematosus, myasthenia gravis, Grave's disease, glomerulonephritis, autoimmune hepatological disorder, inflammatory bowel diseases (e.g., Crohn's disease or ulcerative colitis), anaphylaxis, allergic reaction, Sjögren's syndrome, type I diabetes, primary biliary cirrhosis, Wegener's granulomatosis, fibromyalgia, polymyositis, dermatomyositis, inflammatory myositis, multiple endocrine failure, Schmidt's syndrome, autoimmune uveitis, Addison's disease, adrenalitis, thyroiditis, Hashimoto's thyroiditis, autoimmune thyroid disease, pernicious anemia, gastric atrophy, chronic hepatitis, lupoid hepatitis, atherosclerosis, subacute cutaneous lupus erythematosus, hypoparathyroidism, Dressler's syndrome, autoimmune thrombocytopenia, idiopathic thrombocytopenic purpura, hemolytic anemia, pemphigus vulgaris, pemphigus, dermatitis herpetiformis, alopecia arcata, pemphigoid, scleroderma, progressive systemic sclerosis, CREST syndrome (calcinosis, Raynaud's phenomenon, esophageal dysmotility, sclerodactyly and telangiectasia), male and female autoimmune infertility, ankylosing spondolytis, ulcerative colitis, mixed connective tissue disease, polyarteritis nedosa, systemic necrotizing vasculitis, atopic dermatitis, atopic rhinitis, Goodpasture's syndrome, Chagas' disease, sarcoidosis, rheumatic fever, asthma, recurrent abortion, anti-phospholipid syndrome, farmer's lung, erythema multiforme, post cardiotomy syndrome, Cushing's syndrome, autoimmune chronic active hepatitis, bird fancier's lung, toxic epidermal necrolysis, Alport's syndrome, alveolitis, allergic alveolitis, fibrosing alveolitis, interstitial lung disease, erythema nodosum, pyoderma gangrenosum, transfusion reaction, Takayasu's arteritis, polymyalgia rheumatica, temporal arteritis, schistosomiasis, giant cell arteritis, ascariasis, aspergillosis, Sampter's syndrome, eczema, lymphomatoid granulomatosis, Behcet's disease, Caplan's syndrome, Kawasaki's disease, dengue, encephalomyelitis, endocarditis, endomyocardial fibrosis, endophthalmitis, erythema elevatum diutinum, psoriasis, erythroblastosis fetalis, eosinophilic faciitis, Shulman's syndrome, Felty's syndrome, filariasis, cyclitis, chronic cyclitis, heterochronic cyclitis, Fuch's cyclitis, IgA nephropathy, Henoch-Schonlein purpura, graft-versus-host disease, graft rejection, cardiomyopathy, Eaton-Lambert syndrome, relapsing polychondritis, cryoglobulinemia, Waldenstrom's macroglobulemia, Evan's syndrome, acute respiratory distress syndrome, pulmonary inflammation, osteoporosis, delayed type hypersensitivity and autoimmune gonadal failure. Examples are Sjögren's syndrome, multiple sclerosis and systemic lupus erythematosus.

In some embodiments, provided are a method for treating diseases related to B lymphocytes (e.g., systemic lupus erythematosus, Goodpasture's syndrome, rheumatoid arthritis and type I diabetes), Th1-lymphocytes (e.g., rheumatoid arthritis, multiple sclerosis, psoriasis, Sjögren's syndrome, Hashimoto's disease, Grave's disease, primary biliary cirrhosis, Wegener's granulomatosis, tuberculosis or graft-versus-host disease) or Th2-lymphocytes (e.g., atopic dermatitis, systemic lupus erythematosus, atopic asthma, rhinoconjunctivitis, allergic rhinitis, Omenn's syndrome, systemic sclerosis or chronic graft-versus-host disease), and related pharmaceutical use, the method comprising administering to a subject in need thereof an effective amount of the aforementioned anti-CD40 antibody or antigen-binding fragment thereof.

In some embodiments, provided are a method for treating a tumor or cancer, and related pharmaceutical use, the method comprising administering to a subject in need thereof an effective amount of the aforementioned anti-CD40 antibody or antigen-binding fragment thereof, wherein the tumor or cancer may or may not be related to CD40 expression.

In some embodiments, provided is a method for treating or alleviating graft-versus-host disease or graft rejection, comprising administering to a subject in need thereof the aforementioned anti-CD40 antibody or antigen-binding fragment thereof and tacrolimus. In some embodiments, provided is use of the aforementioned anti-CD40 antibody or antigen-binding fragment thereof for the preparation of a medicament for treating or alleviating graft-versus-host disease or graft rejection, including use in combination with tacrolimus. In some embodiments, provided is use of tacrolimus for the preparation of a medicament for treating or alleviating graft-versus-host disease or graft rejection, including use in combination with the aforementioned anti-CD40 antibody or antigen-binding fragment thereof.

In some embodiments, provided are a method for using the aforementioned anti-CD40 antibody or antigen-binding fragment thereof in combination with tacrolimus to treat or alleviate graft-versus-host disease or graft rejection, and use in combination for the preparation of a medicament for treating or alleviating graft-versus-host disease or graft rejection.

In some embodiments, the graft described above is a solid organ graft, such as a kidney graft, a liver graft, a heart graft, a lung graft, a pancreas graft, a small intestine graft or a composite tissue graft.

In some embodiments, the graft described above refers to grafting one selected from the group consisting of an allogeneic cell, a xenogeneic cell, an allogeneic tissue, a xenogeneic tissue, an allogeneic organ and a xenogeneic organ.

In some embodiments, the aforementioned anti-CD40 antibody or antigen-binding fragment thereof inhibits or reverses the rejection of the tissue graft by the graft acceptor, or prolongs or preserves the function of the tissue grafted into the graft acceptor, or restores the function of the damaged graft tissue in the graft acceptor.

### Detection

The present disclosure provides a composition for detecting CD40, the composition comprising the anti-CD40 antibody or the antigen-binding fragment thereof according to the present disclosure. The present disclosure also provides a method, system or device for detecting CD40 *in vivo* or *in vitro,* the method comprising treating a sample with the anti-CD40 antibody or the antigen-binding fragment thereof of the present disclosure.

In some embodiments, the method, system or device for *in vitro* detection may, for example, comprise:
(1) contacting the sample with the anti-CD4 antibody or the antigen-binding fragment thereof;
(2) detecting formation of a complex between the anti-CD40 antibody or the antigen-binding fragment thereof and the sample; and/or
(3) contacting a reference sample (e.g., a control sample) with the antibody; and
(4) determining the extent of formation of the complex by comparison with the reference sample. A change (e.g., a statistically significant change) in the formation of the complex in the sample or subject as compared to a control sample or subject indicates the presence of CD40 in the sample.

In some other embodiments, the method, system or device for *in vivo* detection may comprise:
(1) administering to a subject the anti-CD40 antibody or the antigen-binding fragment thereof; and
(2) detecting formation of a complex between the anti-CD40 antibody or the antigen-binding fragment thereof and the subject.

The detection may include determining the location or time at which the complex is formed. The CD40 antibody is labeled with a detectable substance, and the label is detected to achieve detection of the substance that binds to the CD40 antibody (e.g., CD40). Suitable detectable substances include various enzymes, prosthetic groups, fluorescent substances, luminescent substances, and radioactive substances. The formation of the complex between the CD40-binding antibody or the antigen-binding fragment thereof and CD40 may be detected by determining or visualizing the antibody that binds to or does not bind to CD40. Conventional detection assays may be used, such as enzyme-linked immunosorbent assay (ELISA), radioimmunoassay (RIA) or tissue immunohistochemistry. For detection purposes, the anti-CD40 antibody or the fragment thereof of the present disclosure may be labeled with a fluorophore chromophore.

In some embodiments, also provided is a kit comprising an anti-CD40 antibody or an antigen-binding fragment thereof, which may also comprise instructions for diagnostic use. The kit may also comprise at least one additional reagent, such as a label or an additional diagnostic agent. For *in vivo* use, the antibody may be formulated into a pharmaceutical composition.

### Definition of Terms

In order to facilitate the understanding of the present disclosure, some technical and scientific terms are specifically defined below. Unless otherwise specifically defined herein, all other technical and scientific terms used herein have the meanings generally understood by those of ordinary skill in the art to which the present disclosure belongs. Unless the context clearly requires otherwise, throughout the description and the claims, the words "comprise", "have", "include", and the like are to be construed in an inclusive sense as opposed to an exclusive or exhaustive sense; that is to say, in the sense of "including, but not limited to".

The three-letter and single-letter codes for amino acids used herein are as described in J. biol. chem, 243, p3558 (1968).

"CD40" and "CD40 antigen" refer to an approximately 48 kD glycoprotein expressed on the surface of normal and neoplastic B cells, which acts as a receptor for signals involved in cellular proliferation and differentiation (Ledbetter et al., 1987, J. Immunol. 138:788-785). A cDNA molecule encoding CD40 has been isolated from a library prepared from the Burkitt lymphoma cell line Raji (Stamenkovic et al., 1989, EMBO J. 8:1403). Sequence information can be found in Table 2 of the present disclosure. A cell that endogenously expresses CD40 is any cell characterized by the surface expression of CD40, including but not limited to, normal and neoplastic B cells, interdigitating cells, basal epithelial cells, carcinoma cells, macrophages, endothelial cells, follicular dendritic cells, tonsil cells, and bone marrow-derived plasma cells.

"Antibody" is used in the broadest sense and encompasses a variety of antibody structures, including, but not limited to monoclonal antibodies, polyclonal antibodies; monospecific antibodies, multispecific antibodies (e.g., bispecific antibodies); and full-length antibodies, and antibody fragments (or antigen-binding fragments, or antigen-binding portions) so long as they exhibit the desired antigen-binding activity. An antibody may refer to an immunoglobulin, a tetrapeptide chain structure formed by linking two identical heavy chains and two identical light chains by interchain disulfide bonds. The heavy chain constant regions of an immunoglobulin differ in their amino acid composition and arrangement, and thus in their antigenicity. Accordingly, immunoglobulins can be divided into five classes, or isotypes of immunoglobulins, namely IgM, IgD, IgG, IgA and IgE, with their corresponding heavy chains being µ chain, δ chain, γ chain, a chain and ε chain, respectively. Ig of the same class can be divided into different subclasses according to differences in the amino acid composition of the hinge regions and the number and positions of disulfide bonds of the heavy chains; for example, IgG can be divided into IgG1, IgG2, IgG3 and IgG4. Light chains are divided into κ or λ chains according to differences in the constant regions. Each of the five classes of Ig may have a κ chain or λ chain. In the antibody heavy and light chains, the sequences of about 110 amino acids near the N-terminus vary considerably and thus are referred to as variable regions (V regions); the remaining amino acid sequences near the C-terminus are relatively stable and thus are referred to as constant regions (C regions). The variable regions comprise 3 hypervariable regions (CDRs) and 4 framework regions (FRs) with relatively conservative sequences. The 3 hypervariable regions determine the specificity of the antibody and thus are also known as complementarity determining regions (CDRs). Each of the light chain variable regions (VLs) and the heavy chain variable regions (VHs) consists of 3 CDR regions and 4 FR regions arranged from the amino terminus to the carboxyl terminus in the following order: FR1, CDR1, FR2, CDR2, FR3, CDR3, and FR4. The 3 CDR regions of the light chain refer to LCDR1, LCDR2, and LCDR3; and the 3 CDR regions of the heavy chain refer to HCDR1, HCDR2, and HCDR3.

For the determination or definition of "CDRs", the deterministic depiction of CDRs and identification of residues comprising antigen-binding sites of the antibody can be accomplished by resolving the structure of the antibody and/or resolving the structure of the antibody-ligand complex. This can be accomplished by any of a variety of techniques known to those skilled in the art, such as X-ray crystallography. A variety of analysis methods can be used to identify CDRs, including but not limited to the Kabat numbering scheme, the Chothia numbering scheme, the AbM numbering scheme, the IMGT numbering scheme, the contact definition, and the conformational definition.

The Kabat numbering scheme is a standard for numbering residues in antibodies and is generally used to identify CDR regions (see, e.g., Johnson & Wu, 2000, Nucleic Acids Res., 28:214-8). The Chothia numbering scheme is similar to the Kabat numbering scheme, except that it takes into account the position of certain structural loop regions. (see, e.g., Chothia et al., 1986, J. Mol. Biol., 196:901-17; Chothia et al., 1989, Nature, 342:877-83). The AbM numbering scheme adopts a computer program integration suite for modeling antibody structures manufactured by Oxford Molecular Group (see, e.g., Martin et al., 1989, Proc Natl Acad Sci (USA), 86:9268-9272; "AbMTM, A Computer Program for Modeling Variable Regions of Antibodies", Oxford, UK; Oxford Molecular, Ltd.). The AbM numbering scheme adopts a combination of a knowledge database and the de-novo method to model the tertiary structure of antibodies from basic sequences (see those described in Samudrala et al., 1999, "Ab Initio Protein Structure Prediction Using a Combined Hierarchical Approach", PROTEINS, Structure, Function and Genetics Suppl., 3:194-198). The contact definition is based on the analysis of the available complex crystal structures (see, e.g., MacCallum et al., 1996, J. Mol. Biol., 5:732-45). In the conformational definition, the positions of the CDRs can be identified as residues that contribute enthalpy to the antigen binding (see, e.g., Makabe et al., 2008, Journal of Biological Chemistry, 283:1156-1166). In addition, other CDR boundary definitions may not strictly follow one of the above methods, but still overlap with at least a portion of the Kabat CDRs, although they may be shortened or lengthened based on predictions or experimental results that a particular residue or a particular group of residues do not significantly affect the antigen binding. As used herein, a CDR may refer to a CDR defined by any method known in the art, including combinations of methods. The correspondence between the various numbering schemes is well known to those skilled in the art, and examples are shown in Table 1 below.

**Table 1. Relationships between CDR numbering schemes**

| CDR | IMGT | Kabat | AbM | Chothia | Contact |
|---|---|---|---|---|---|
| HCDR1 | 27-38 | 31-35 | 26-35 | 26-32 | 30-35 |
| HCDR2 | 56-65 | 50-65 | 50-58 | 52-56 | 47-58 |
| HCDR3 | 105-117 | 95-102 | 95-102 | 95-102 | 93-101 |
| LCDR1 | 27-38 | 24-34 | 24-34 | 24-34 | 30-36 |
| LCDR2 | 56-65 | 50-56 | 50-56 | 50-56 | 46-55 |
| LCDR3 | 105-117 | 89-97 | 89-97 | 89-97 | 89-96 |

The CDR amino acid residues of the VL and VH regions of the antibody or the antigen-binding fragment of the present disclosure accord with the known Kabat numbering scheme in terms of number and positions.

"Monoclonal antibody" or "mAb" refers to an antibody obtained from a population of substantially homogeneous antibodies, i.e., the individual antibodies included in the population are identical except for possible naturally-occurring mutations that may be present in minor amounts. Monoclonal antibodies are highly specific, being directed against a single antigenic site. Furthermore, in contrast to polyclonal antibody preparations, which typically include different antibodies directed against different determinants (epitopes), each monoclonal antibody is directed against a single determinant on the antigen. The modifier "monoclonal" indicates the characteristics of an antibody obtained from a population of substantially homogeneous antibodies, and is not to be construed as requiring production of the antibody by any particular method.

The term "rabbit antibody" herein refers to a monoclonal antibody directed against human CD40 or an epitope thereof prepared according to the knowledge and skill in the art. In the preparation, a test rabbit is injected with the CD40 antigen, and then antibodies expressed with the desired sequences or functional properties are isolated. In one specific embodiment of the present disclosure, the rabbit anti-human CD40 antibody or the antigen-binding fragment thereof may further comprise a light chain constant region of a rabbit κ or λ chain or a variant thereof, or further comprise a heavy chain constant region of a rabbit IgG1, IgG2, IgG3 or IgG4 or a variant thereof.

The term "fully human antibody" includes antibodies having variable and constant regions derived from human germline immunoglobulin sequences. The fully human antibody of the present disclosure may include amino acid residues not encoded by human germline immunoglobulin sequences (e.g., mutations introduced by random or site-specific mutagenesis *in vitro* or by somatic mutations *in vivo*)*.* However, the term "fully human antibody" does not include antibodies in which CDR sequences derived from the germline of another mammalian species (such as rabbits) have been grafted into human framework sequences (i.e., "humanized antibody").

The term "humanized antibody", also known as a CDR-grafted antibody, refers to an antibody produced by grafting non-human CDR sequences into the framework of variable regions of a human antibody. Such an antibody can overcome the strong immune response induced by the chimeric antibody because of carrying a large number of non-human protein components. To avoid the decrease in activity caused by the decrease in immunogenicity, the variable regions of a fully human antibody can be subjected to minimum reverse mutation to maintain activity.

The term "chimeric antibody" refers to an antibody obtained by fusing variable regions of an antibody of a first species to constant regions of an antibody of a second species, which can reduce an immune response induced by the antibody of the first species. As an example, the chimeric antibody is established by firstly establishing rabbits secreting a rabbit specific monoclonal antibody, isolating the antibody, then cloning a constant region gene of fully human antibody as required, linking the rabbit variable region gene and the human constant region gene into a chimeric gene, inserting the chimeric gene into a human vector, and finally expressing chimeric antibody molecules in a eukaryotic industrial system or prokaryotic industrial system. The constant region of the fully human antibody may be selected from the group consisting of the heavy chain constant regions of human IgG1, IgG2, IgG3 and IgG4 or variants thereof, preferably comprising human IgG1 or IgG4 heavy chain constant regions, or IgG1 mutated at amino acids without ADCC (antibody-dependent cell-mediated cytotoxicity) toxicity.

The term "antigen-binding fragment" includes a single-chain antibody (i.e., full-length heavy and light chains); a Fab, a modified Fab, a Fab', a modified Fab', an F(ab')2, an Fv, a Fab-Fv, a Fab-dsFv, a single domain antibody (e.g., VH or VL or VHH), an scFv, a bivalent or trivalent or tetravalent antibody, a Bis-scFv, a diabody, a tribody, a triabody, a tetrabody and an epitope-binding fragment of any of the above (see, e.g., Holliger and Hudson, 2005, Nature Biotech. 23(9): 1126-1136; Adair and Lawson, 2005, Drug Design Reviews-Online 2(3), 209-217). Methods for producing and preparing such antibody fragments are well known in the art (see, e.g., Verma et al., 1998, Journal of Immunological Methods, 216, 165-181). Fab-Fv was first disclosed in WO2009/040562, and its disulfide-stabilized form Fab-dsFv was first disclosed in WO2010/035012. The antigen-binding fragment of the present disclosure also includes Fab and Fab' fragments described in WO2005/003169, WO2005/003170 and WO2005/003171. Multivalent antibodies may comprise multiple specificities (e.g., bispecificites) or may be monospecific (see, e.g., WO92/22583 and WO05/113605), and an example of the latter is Tri-Fab (or TFM) described in WO92/22583.

The term "bind to CD40" refers to being able to interact with CD40 or an epitope thereof, wherein the CD40 or the epitope thereof may be derived from humans. The term "antigen-binding site" herein refers to a discontinuous three-dimensional spatial site on an antigen that is recognized by the antibody or the antigen-binding fragment of the present disclosure.

The term "antigen" refers to a molecule used for immunization of an immunocompetent vertebrate to produce an antibody that recognizes the antigen or to screen an expression library (e.g., particularly phage, yeast or ribosome display library). Herein, the antigen is defined in a broader sense, and includes a target molecule that is specifically recognized by the antibody, and a portion or a mimic of a molecule used in an immunization process for producing the antibody or in library screening for selecting the antibody. For the antibody of the present disclosure that binds to human CD40, monomers and polymers (e.g., dimers, trimers, etc.) of human CD40, and truncated variants and other variants of human CD40 are all referred to as antigens.

The term "epitope" refers to a site on an antigen to which an immunoglobulin or an antibody binds. An epitope may be formed from contiguous amino acids, or non-contiguous amino acids juxtaposed by tertiary folding of the protein. An epitope formed from contiguous amino acids is generally retained after exposure to a denaturing solvent, while an epitope formed by tertiary folding is generally lost after a denaturing solvent treatment. An epitope generally comprises, for example, at least 3-15 amino acids in a unique spatial conformation. Methods for determining what epitope is bound by a given antibody are well known in the art and include an immunoblotting assay, an immunoprecipitation assay, and the like. Methods for determining the spatial conformation of an epitope include techniques in the art and techniques described herein, such as X-ray crystallography and two-dimensional nuclear magnetic resonance.

The term "specific binding" or "selective binding" refers to binding of an antibody to an epitope on a predetermined antigen. Generally, an antibody binds to a predetermined antigen or epitope thereof with an equilibrium dissociation constant (K_{D}) of about less than 10⁻⁷ M or even less and with an affinity that is at least twice as high as its affinity for binding to a non-specific antigen other than the predetermined antigen or the epitope thereof (or non-specific antigens other than closely related antigens, e.g., BSA, etc.), when determined by surface plasmon resonance (SPR) techniques in an instrument using human CD40 or an epitope thereof as an analyte and the antibody as a ligand. The term "antigen-recognizing antibody" is used interchangeably herein with the term "specifically bound antibody".

"Binding affinity" or "affinity" is used herein as a measure of the strength of a noncovalent interaction between two molecules (e.g., an antibody or a portion thereof and an antigen). The binding affinity between two molecules can be quantified by determining the dissociation constant (K_{D}). KD can be determined by measuring the kinetics of complex formation and dissociation using, for example, the surface plasmon resonance (SPR) method (Biacore). The rate constants corresponding to the association and dissociation of a monovalent complex are referred to as the association rate constant ka (or kon) and the dissociation rate constant kd (or koff), respectively. K_{D} is related to ka and kd by the equation K_{D} = kd/ka. The value of the dissociation constant can be determined directly by well-known methods and can be calculated by methods such as those described by Caceci et al (1984, Byte 9:340-362) even for complex mixtures. For example, K_{D} can be determined by using a dual filtration nitrocellulose filter binding assay such as that disclosed by Wong & Lohman (1993, Proc. Natl. Acad. Sci. USA 90:5428-5432). Other standard assays for evaluating the binding ability of an antibody to a target antigen are known in the art and include, for example, ELISA, western blot, RIA and flow cytometry, as well as other assays exemplified elsewhere herein. The binding kinetics and binding affinity of the antibody can also be evaluated by standard assays known in the art, such as surface plasmon resonance (SPR), for example, by using the Biacore^{™} system or KinExA. The binding affinities associated with different molecular interactions, e.g., the binding affinities of different antibodies for a given antigen, can be compared by comparing the K_{D} values of antibody/antigen complexes. Similarly, the specificity of an interaction can be evaluated by determining and comparing the K_{D} value for the interaction of interest (e.g., a specific interaction between an antibody and an antigen) with the K_{D} value for an interaction not of interest (e.g., a control antibody known not to bind to CD40).

The term "conservative replacement" refers to replacement by another amino acid residue having similar properties to the original amino acid residue. For example, lysine, arginine and histidine have similar properties in that they have basic side chains, and aspartic acid and glutamic acid have similar properties in that they have acidic side chains. In addition, glycine, asparagine, glutamine, serine, threonine, tyrosine, cysteine and tryptophan have similar properties in that they have uncharged polar side chains, and alanine, valine, leucine, threonine, isoleucine, proline, phenylalanine and methionine have similar properties in that they have nonpolar side chains. In addition, tyrosine, phenylalanine, tryptophan and histidine have similar properties in that they have aromatic side chains. Thus, it will be apparent to those skilled in the art that even when an amino acid residue in a group exhibiting similar properties as described above is replaced, it will not exhibit a particular change in properties.

The terms "inhibition" and "blocking" are used interchangeably and encompass both partial and complete inhibition/blocking. Inhibition/blocking of CD40 preferably reduces or alters the normal level or type of activity that occurs when CD40 binding occurs without inhibition or blocking. Inhibition and blocking are also intended to include any measurable decrease in CD40 binding affinity when in contact with an anti-CD40 antibody as compared to CD40 not in contact with an anti-CD40 antibody.

"Inhibition of growth" (e.g., involving cells) is intended to include any measurable reduction in cell growth.

"Agonistic activity", "agonist activity" or "agonism" refers to the function as an agonist. The binding of an agonist to a cell receptor initiates a reaction or activity that is similar to or the same as that initiated by the receptor's natural ligand. For example, a CD40 agonist can induce any or all of the following responses: cell proliferation and/or differentiation; up-regulation of intercellular adhesion via such molecules as ICAM-1, E-selectin and VCAM; secretion of pro-inflammatory cytokines such as IL-1, IL-6, IL-8, IL-12 and TNF; signal transduction through the CD40 receptor by such pathways as TRAF (e.g., TRAF2 and/or TRAF3), MAP kinases such as NIK (NF-κB inducing kinase), 1-κB kinases (IKKα/β), transcription factor NF-xB, Ras and the MEK/ERK pathway, the PI3K/Akt pathway, and the P38 MAPK pathway; transduction of anti-apoptotic signals by such molecules as XIAP, Mcl-1 and BCLx; B and/or T cell memory generation: B cell antibody production; B cell isotype switching; up-regulation of cell surface expression of MHC class II and CD80/86, and the like. "Antagonistic activity", "antagonist activity" or "antagonism" refers to the function of a substance as an antagonist. For example, an antagonist of CD40 can prevent or reduce any of the responses induced by the binding of the CD40 receptor to an agonist ligand, particularly CD40L. The antagonist may reduce any one or more of the responses induced by agonist binding by 5%, 10%, 15%, 20%, 25%, 30%, 35%, preferably 40%, 45%, 50%, 55%, 60%, more preferably 70%, 80%, 85%, and most preferably 90%, 95%, 99% or 100%. Methods for measuring anti-CD40 antibody and CD40-ligand binding specificity and antagonist activity are known to those skilled in the art and include, but are not limited to, standard competitive binding assays, assays for monitoring immunoglobulin secretion by B cells, B cell proliferation assays, Banchereau-like B cell proliferation assays, T cell helper assays for antibody production, co-stimulation of B cell proliferation assays, and assays for up-regulation of B cell activation markers.

Methods for producing and purifying antibodies and antigen-binding fragments are well known in the prior art and can be found in, for example, "Antibodies: A Laboratory Manual", Cold Spring Harbor Press (chapters 5-8 and 15). For example, mice or rabbits can be immunized with human CD40 or a fragment thereof, and the resulting antibodies can be renatured and purified, and amino acid sequencing can be performed by conventional methods. Likewise, antigen-binding fragments can be prepared by conventional methods. The antibody or the antigen-binding fragment described herein is genetically engineered to contain one or more human FR regions in the non-human CDR regions. Human FR germline sequences are available from the ImMunoGeneTics (IMGT) website.

Antibodies can be competitively screened for binding to the same epitope using conventional techniques known to those skilled in the art. For example, competition and cross-competition studies can be performed to obtain antibodies that compete or cross-compete with one another for binding to an antigen. A high-throughput method for obtaining antibodies that bind to the same epitope based on their cross-competition is described in International Patent Publication No. WO03/48731. Therefore, an antibody and an antigen-binding fragment thereof that compete for binding to the same epitope on CD40 with the antibody molecule of the present disclosure can be obtained by conventional techniques known to those skilled in the art.

The term "disorder" is any condition that would benefit from treatment with a humanized anti-CD40 antibody of the present disclosure. This includes chronic and acute disorders or diseases. Non-limiting examples of disorders to be treated by the present disclosure include cancer, hematological malignancies, benign and malignant tumors, leukemias and lymphoid malignancies and inflammatory, angiogenic, and autoimmune and immunological disorders.

The term "CD40-related disorder" or "CD40-related disease" refers to a condition in which modification or elimination of cells expressing CD40 is indicated. These cells include CD40-expressing cells demonstrating abnormal proliferation or CD40-expressing cells that are associated with cancerous or malignant growth. More specific examples of cancers that demonstrate abnormal expression of the CD40 antigen include B lymphoblastoid cells, Burkitt's lymphoma, multiple myeloma, T cell lymphomas, Kaposi's sarcoma, osteosarcoma, epidermal and endothelial tumors, pancreatic cancer, lung cancer, breast cancer, ovarian cancer, colon cancer, prostate cancer, head and neck cancer, skin cancer (melanoma), bladder cancer, and kidney cancer. Such disorders include, but are not limited to, leukemias, lymphomas (including B cell lymphoma and non-Hodgkin lymphoma), multiple myeloma, Waldenstrom's macroglobulinemia; solid tumors, including sarcomas, such as osteosarcoma, Ewing's sarcoma, malignant melanoma, adenocarcinoma (including ovarian adenocarcinoma), Kaposi's sarcomalKaposi's tumor and squamous cell carcinoma. "CD40-related disorders" also include diseases and disorders of the immune system, such as autoimmune disorders and inflammatory disorders. Such conditions include, but are not limited to, rheumatoid arthritis (RA), systemic lupus erythematosus (SLE), scleroderma, Sjögren's syndrome, multiple sclerosis, psoriasis, inflammatory bowel diseases (e.g., ulcerative colitis and Crohn's disease), pulmonary inflammation, asthma, and idiopathic thrombocytopenic purpura (ITP).

The term "arrests the growth of" or "growth inhibition" refers to inhibiting the growth or proliferation of a cell, especially a neoplastic cell type expressing the CD40 antigen. Thus, growth inhibition, for example, significantly reduces the percentage of neoplastic cells in S phase.

"Giving", "administering", and "treating", when applied to animals, humans, experimental subjects, cells, tissues, organs or biological fluid, refer to contact of an exogenous drug, a therapeutic agent, a diagnostic agent or a composition with the animals, humans, subjects, cells, tissues, organs or biological fluid. "Giving", "administering", and "treating" can refer to, for example, therapeutic, pharmacokinetic, diagnostic, research, and experimental methods. The treatment of cells comprises contacting the reagent with the cells and contacting the reagent with fluid, where the fluid is in contact with the cells. "Giving", "administering", and "treating" also refer to treating, e.g., a cell, by a reagent, diagnosis, a binding composition, or by another cell *in vitro* and *ex vivo.* "Treating", when applied to humans, veterinary or research subjects, refers to therapeutic treatment, preventive or prophylactic measures, and research and diagnostic applications.

"Treatment" refers to administering a therapeutic agent, such as any of the antibodies or the antigen-binding fragments thereof of the present disclosure or a composition comprising same, either internally or externally to a subject who has had, is suspected of having, or is predisposed to having one or more diseases or symptoms thereof on which the therapeutic agent is known to have a therapeutic effect. Generally, the therapeutic agent is administered in an amount effective to alleviate one or more symptoms of the disease in the subject or population being treated, whether by inducing regression of such symptoms or inhibiting the development of such symptoms into any clinically measurable degree. The amount of therapeutic agent effective to alleviate any particular symptom of the disease (also referred to as the "therapeutically effective amount") may vary depending on factors such as the disease state, age and weight of the subject, and the ability of the drug to produce a desired therapeutic effect in the subject. Whether a symptom of a disease has been alleviated can be evaluated by any clinical testing methods commonly used by doctors or other health care professionals to evaluate the severity or progression of the symptom. Although embodiments of the present disclosure (e.g., treatment methods or articles of manufacture) may be ineffective in alleviating symptoms of a disease of interest in a certain subject, they shall alleviate the symptoms of the disease of interest in a statistically significant number of subjects as determined by any statistical test method known in the art, such as the Student's t-test, Chi-square test, U-test by Mann and Whitney, Kruskal-Wallis test (H-test), Jonckheere-Terpstra test and Wilcoxon test.

"Effective amount" comprises an amount sufficient to ameliorate or prevent a symptom or sign of a medical condition. An effective amount also refers to an amount sufficient to allow or facilitate diagnosis. The effective amount for a particular subject or veterinary subject may vary depending on factors such as the condition to be treated, the general health of the subject, the method and route and dosage of administration, and the severity of side effects. An effective amount may be the maximum dose or administration regimen to avoid significant side effects or toxic effects.

"Homology" or "identity" refers to sequence similarity between two polynucleotide sequences or between two polypeptides. When positions in two compared sequences are occupied by identical nucleotides or amino acid monomer subunits, e.g., if the position of each of two DNA molecules is occupied by an identical nucleotide, the molecules are homologous at that position. The homology percentage between two sequences is a function of the number of matching or homologous positions shared by the two sequences divided by the number of positions compared × 100%. For example, if 6 out of 10 positions are matched or homologous when two sequences are optimally aligned, the two sequences are 60% homologous. Generally, when two sequences are aligned, comparison is performed to obtain the maximum homology percentage.

"Cell", "cell line", and "cell culture" are used interchangeably, and all such designations include their progenies. It should also be understood that all progenies may not be precisely identical in DNA content due to intentional or unintentional mutations. Mutant progeny with identical function or biological activity as screened in the original transformed cells is included.

"Optional" or "optionally" means that the event or circumstance subsequently described may, but does not necessarily, occur, and that the description includes instances where the event or circumstance occurs or does not occur. For example, "optionally comprising 1-3 antibody heavy chain variable regions" means that the antibody heavy chain variable region of a particular sequence may, but does not necessarily, exist.

The "CD40-binding protein" of the present disclosure is interpreted in a maximized sense, and includes the anti-CD40 antibody or the antigen-binding fragment thereof of the present disclosure, and any protein capable of achieving binding to CD40 is within the scope of the term. For example, a CD40-binding protein may comprise one or more effector molecules, e.g., in the form of a conjugate. The "effector molecule" alone may be therapeutically active (e.g., have anti-tumor activity or immune-activating or inhibiting activity) or have a detecting function, and may be in any form, such as biologically active proteins (e.g. enzymes), other antibody or antibody fragments, synthetic or naturally occurring polymers, polynucleotides and fragments thereof (DNA and RNA and fragments thereof), radionuclides (particularly radioiodides), radioisotopes, chelated metals, nanoparticles and reporter groups (e.g., fluorescent compounds) or compounds that can be detected by NMR or ESR spectroscopy. Conjugation of the effector molecule to the anti-CD40 antibody or the antigen-binding fragment thereof of the present disclosure can be achieved by conventional methods.

"Antibody-drug conjugate" refers to a conjugate formed by linking a drug (e.g., an antineoplastic agent or a toxin) to an antibody, which can be achieved by conventional methods, e.g., by a cleavable or non-cleavable linker.

### BRIEF DESCRIPTION OF THE DRAWINGS

FIGs. 1A to 1B: the activity of CD40 antagonistic antibodies in a reporter gene system.

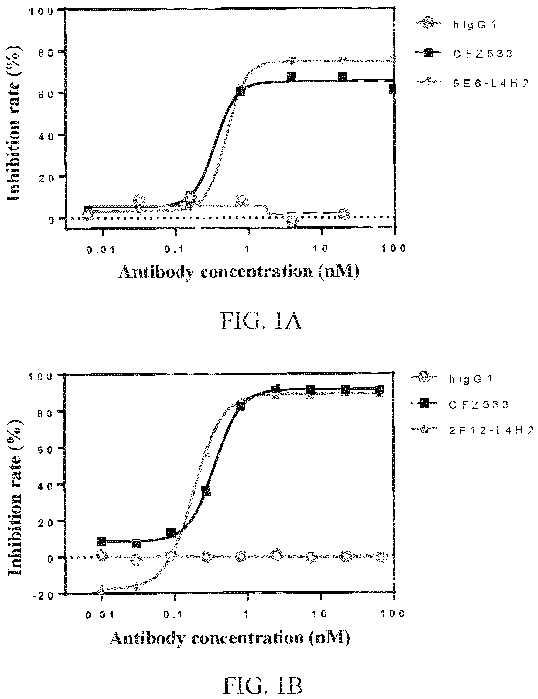

FIGs. 1A and 1B show the related results for 9E6-L4H2 and 2F12-L4H2, respectively.
FIGs. 1A and 1B both use a human IgG1 isotype as a negative control and CFZ533 as a positive control.
FIGs. 2A to 2B: the inhibitory activity of CD40 antagonistic antibodies in a B cell activation assay system. FIG. 2A shows the percent inhibition of CD19+CD69+ cells by 9E6-L4H2 and 2F12-L4H2. FIG. 2B shows the MFI inhibition of CD19+CD69+ cells by 9E6-L4H2 and 2F12-L4H2. FIGs. 2A-2B both use a human IgG1 isotype as a negative control and CFZ533 as a positive control.
FIGs. 3A to 3D: the inhibitory activity of CD40 antagonistic antibodies in a DC cell activation assay system. FIG. 3A shows the MFI inhibition of CD11C+CD80+ cells by 9E6-L4H2 and 2F12-L4H2. FIG. 3B shows the MFI inhibition of CD11C+CD86+ cells by 9E6-L4H2 and 2F12-L4H2. FIG. 3C shows the inhibition of IL-12/23 p40 by 9E6-L4H2 and 2F12-L4H2. FIG. 3D shows the inhibition of TNFα by 9E6-L4H2 and 2F12-L4H2. FIGs. 3A-3D all use a human IgG1 isotype as a negative control and CFZ533 as a positive control.
FIG. 4: the endogenous agonistic activity of the CD40 antagonistic antibodies 9E6-L4H2 and 2F12-L4H2 in a B cell activation assay system, with a human IgG1 isotype, CFZ533 and the agonistic anti-CD40 antibody 9E5-SELFNS as controls.
FIGs. 5A to 5B: the activity of CD40 antagonistic antibodies in a mouse T cell-dependent humoral immune response model. FIG. 5A is a flowchart. FIG. 5B is a graph showing the results of the tests on days 7, 14, 21 and 28.
FIGs. 6A to 6B: the activity of CD40 antagonistic antibodies in a mouse skin graft rejection model. FIG. 6A is a flowchart. FIG. 6B shows skin graft survival rates (%) and skin graft scores.
FIG. 7: the activity of combinations of CD40 antagonistic antibodies with tacrolimus (FK506) in a mouse skin graft rejection model. A in FIG. 7 shows the skin graft survival rates (%) for 9E6-L4H2 (10 mpk) and its combination with tacrolimus (FK506). B in FIG. 7 shows the skin graft survival rates (%) for 2F12-L4H2 (10 mpk) and its combination with tacrolimus (FK506). C in FIG. 7 shows the skin graft scores for 9E6-L4H2 (10 mpk) and its combination with tacrolimus (FK506). D in FIG. 7 shows the skin graft scores for 2F12-L4H2 (10 mpk) and its combination with tacrolimus (FK506).
FIG. 8: the PK results of the CD40 antagonistic antibodies 9E6-L4H2 and 2F12-L4H2 in human CD40 transgenic mice, with CFZ533 as a control.
FIGs. 9A to 9B: the inhibitory activity of Fc-mutation CD40 antagonistic antibodies in a B cell activation assay system. FIG. 9A shows the MFI inhibition of CD19+CD69+ cells by 9E6-L4H2 and 9E6-L4H2-AAYTE. FIG. 9B shows the MFI inhibition of CD19+LD69+ cells by 2F12-L4H2 and 2F12-L4H2-AAYTE.
FIGs. 10A to 10D: the inhibitory activity of Fc-mutation CD40 antagonists in a DC cell activation assay system. FIG. 10A shows the inhibition of IL-12/23 p40 by 9E6-L4H2 and 9E6-L4H2-AAYTE. FIG. 10B shows the inhibition of TNFα by 9E6-L4H2 and 9E6-L4H2-AAYTE. FIG. 10C shows the inhibition of IL-12/23 p40 by 2F12-L4H2 and 2F12-L4H2-AAYTE. FIG. 10D shows the inhibition of TNFα by 2F12-L4H2 and 2F12-L4H2-AAYTE.

### DETAILED DESCRIPTION

The present disclosure is further described below with reference to examples, but these examples are not intended to limit the scope of the present disclosure.

Experimental procedures without specific conditions indicated in the examples or test examples are generally conducted according to conventional conditions, or according to conditions recommended by the manufacturer of the starting materials or commercial products. See Sambrook et al., Molecular Cloning: A Laboratory Manual, Cold Spring Harbor Laboratory Press; Current Protocols in Molecular Biology, Ausubel et al., Greene Publishing Association, Wiley Interscience, NY. Reagents without specific origins indicated are commercially available conventional reagents.

### Example 1. CD40 Immunizing Antigens, Sequences for Antigen Screening and Preparation

The his-tagged human CD40 (h-CD40-his) recombinant protein (Cat. # CD0-H5228), the mouse Fc-tagged human CD40 (h-CD40-mFc) recombinant protein (Cat. # CD0-H525a), the his- and biotin-tagged human CD40 (hCD40-his-avi) recombinant protein (Cat. # CD0-H82E8) and the his-tagged cynomolgus monkey CD40 (cyno-CD40-his) recombinant protein (Cat. # CD0-C52H6) were all purified commercial protein reagents purchased from Acrobiosystems Inc., and the sources of their respective sequences are shown in Table 2. The protein reagents can be used in the experiments of the following examples.

**Table 2. The sources of the amino acid sequences of recombinant proteins**

| Name | Start and end of amino acid sequence | GenBank accession No. |
|---|---|---|
| h-CD40-his | Glu21-Arg193 | P25942-1 |
| h-CD40-mFc | Glu21-Arg193 | P25942-1 |
| h-CD40-his-avi | Glu21-Arg193 | P25942-1 |
| cyno-CD40-his | Glu21-Arg193 | G7PG38 |

### Example 2. Screening for Anti-CD40 Rabbit Monoclonal Antibodies and Preparation of Human-Rabbit Chimeric Antibodies

Anti-human CD40 monoclonal antibodies were produced by immunizing 2 New Zealand white rabbits. The immunizing antigen was a His-tagged human CD40 recombinant protein (h-CD40-his, prepared into 1 µg/µL with phosphate buffer). Emulsification with Freund's adjuvants: complete Freund's adjuvant (CFA) was used for the first immunization, and incomplete Freund's adjuvant (IFA) was used for the booster immunizations. For each immunization, a multiple-site subcutaneous injection of 400 µg of the antigen was used. The injections for immunization were performed on days 0, 7, 20 and 41. Blood was collected on days 27 and 48 and tested, and antibody titers in rabbit serum were determined by ELISA and FACS. Rabbits in which the antibody titers were high in serum and tended to stabilize were selected, and each was intravenously injected with 400 µg of an antigen solution prepared with phosphate buffer on day 63 for boost immunization. On day 67, the spleens of these rabbits were collected, and a biotin-tagged CD40 antigen was added. Labeled monoclonal B cells were sorted into a 96-well plate using a flow cytometer. After 14 days of culture, supernatants were collected and screened by ELISA and FACS for clones that could bind to human CD40, cynomolgus monkey CD40 and Raji cells (a tumor cell line that expresses human CD40), and a total of 28 strains of B cell monoclones were obtained. The RNAs of these monoclonal cells were extracted and reverse-transcribed, and after PCR amplification, the products were sent to a sequencing company for sequencing. Finally, the sequences of 28 rabbit antibodies were obtained. These antibodies were screened by affinity and activity assays (see Examples 2-3), and 11 monoclonal antibodies were obtained. Their heavy and light chain variable region sequences are shown in Table 3, and their CDR sequences are shown in Table 4.

**Table 3. The variable region sequences of anti-CD40 rabbit monoclonal antibodies**

| No. | Heavy chain variable region and light chain variable region sequences | |
|---|---|---|
| 5A9 | VH | QSVKESEGGLFKPTDTLTLTCTASRFSLSSYDMSWVRQAPGNGLEWIGAIGGA GGTYYASWAKSRSTITRNTNLNTLTLKMTSLTAADTATYFCARGWTRLDLWG QGTLVTVSS (SEQ ID NO:1) |
| | VL | ADIVLTQTASPVSGAVGGTVTINCQSSEDISSNLSWYQQKPGQPPKLLIYAASN LASGVPSRFKGSGSGTEFTLTISDLECADAATYYCQGGYWSGISNFGNGFGGG TEVVVK (SEQ ID NO:2) |
| 9E6 | VH | QSVRESEGGLVKPTDTLTLTCTVSGFSLSSYDMSWVRQAPGNGLEWIGAIGGA GGTYYASWAKSRSTITRNTNLNTVTLKMSSLTAADTATYFCARGWTRLDLWG QGTLVTVSS (SEQ ID NO:3) |
| | VL | ADIVLTQTPSPVSGAVGGTVTIKCQASEDISSNLAWYQQKPGQPPKLLIFPASNL ASGVSSRFKGSGSGTEFTLTISDLECADAATYYCQGGYWTSTSNFGNGFGGGT EVVVK (SEQ ID NO:4) |
| 9F10 | VH | QSVKESEGGLFKPTDTLTLTCTVSGFSLSSYDMSWVRQAPGNGLEWIGAIGGA GGTYYASWAKSRSTITRNTNLNTVTLKMTSLTAADTATYFCTRGWTRLDLWG QGTLVTVSS (SEQ ID NO:5) |
| | VL | ADIVLTQTESPVSGPVGGTVTINCQASEDISSNLAWYQQKPGQPPKLLIYAASN LASGVPSRFKGSGSGTEFTLTISDLECADAATYYCQGAYWSSTSYFGNGFGGG TQVVVK (SEQ ID NO:6) |
| 4F6 | VH | QSVKESEGGLFKPADTLTLTCTVSRFSLSSYGVTWVRQAPGNGLEWIGAIGST GSAYYASWAKSRSTITRDTNLNTVTLKMTSLTAADTATYFCARGGITAYAIWGP GTLVTVSS (SEQ ID NO:7) |
| | VL | AFELTQTPSSVEAAVGGTVTIKCQASQSISNGLAWYQQKPGQPPKLLIAGASNL ASGVSSRFKGSGSGIEFILTISDLECADAATYYCQSYYNSFTTVFGGGTEVVVK (SEQ ID NO:8) |
| 5B8 | VH | QSVKESEGGLFKPTDTLTLTCTVSGFSLNSYGVSWVRQAPGNGLEWIGAIGSS GSAYYASWARSRSTITRDTNLNTVTLKMTSLTAADTATYFCARGGITVYAIWG PGTLVTVSS (SEQ ID NO:9) |
| | VL | AFELTQTPSPVSAAVGGTVTINCQASEDITNGIAWYQQKPGQPPKLLIAGASNL ASGVSSRFKGSGSGTEFTLTISDLECDDAATYYCQSYYSSSYTIFGGGTEVVVK (SEQ ID NO:10) |
| 8010 | VH | QSVKESEGGLFKPTATLTLTCTVSGFSLSSYGVSWVRQAPGSGLEWIGGIASTG TTYYANWAKSRSTITRDTNLKTVTLKMTSLTAADTATYFCARGGITAYAIWGP GTLVTVSS (SEQ ID NO:11) |
| | VL | AFELTQTPSSVEAAVGGTVTIKCQASQSITNGLAWYQQKPGQPPKLLIAGASNL ASGVSSRFKGSGSGTEFTLTISDLECADAATYYCQSYYDSSSTVFGGGTEVVV K (SEQ ID NO:12) |
| 8C12 | VH | QSVKESEGGLFKPTDTLTLTCTVSGFSLSSYGVSWVRQAPGNGLEWIGGIGSD GSAYYASWAKSRATITRDTNLKTVTLEMTSLTVADTATYFCARGGITVYAIWGP GTLVTVSS (SEQ ID NO:13) |
| | VL | AFELTQTPSPVSAAVGGTVTIKCQASQSISNGLAWYQQKPGQPPKVLIVGASNL ASGVSSRFKGSGSGTEFTLSISDLECADGATYYCQSYFSSSSTVFGGGTEVVVK (SEQ ID NO:14) |
| 4D4 | VH | QSVKESEGGLFKPTDTLTLTCTVSGFSLSSYGVSWVRQAPGNGLEWIGGIGSD GSAYYASWAKSRATITRDTNLKTVTLEMTSLTAADTATYFCARGGITVYAIWG PGTLVTVSS (SEQ ID |
| | | NO:15) |
| | VL | AFELTQTPSPVSAAVGGTVTIKCQASQSISNGLAWYQQKPGQPPKVLIVGASNL ASGVSSRFKGSGSGTEFTLSISDLECADGATYYCQSYFSSSSTVFGGGTEVVVK (SEQ ID NO:16) |
| 2F12 | VH | QSVKESEGGLFKPKDTLTLTCTVSGFSLSSYGVSWVRQAPGNGLEWIGGIGSD GSAYYASWAKSRATITRDTNLKTVTLEMTSLTAADTATYFCARGGITVYAMW GPGTLVTVSS (SEQ ID NO:17) |
| | VL | AFELTQTPASVEAAMGGTVTIKCQASQSISNGLAWYQQKPGQPPKLLIVGASN LASGVSSRFKGSGSGTEFTLSISDLECADGATYYCQSYFSSSSTVFGGGTEVVV K (SEQ ID NO:18) |
| 3F6 | VH | QSVKESEGGLFKPTDTLTLTCTVSGFSLSSYAISWVRQAPGNGLEWIGAIDRYG TTYYATWAKSRSTITRNTNENTVTLKMTSLTAADTATYFCARGPWYYGGDVA WTGSFDPWGPGTLVTVSS (SEQ ID NO:19) |
| | VL | [?] AQVLTQTASPVSAAVGGTVTISCQSSQSVANNDFLSWYQQKPGQPPKLLIYGA STLASGVPSRFRGNGSGTQFTLTITGMQCDDAATYFCTGGYAGPIYIFGGGTEV VVK (SEQ ID NO:20) |
| 10A4 | VH | QSLEESGGRLVTPGGSLTLTCTVSGIDLSRNAISWVRQSPGNGLEWIGGIGSSGS AYYASWAKSRSTITRDTNLNTVTLKMTSLTAADTATYFCARDGYAGSSWGIYY GMDPWGPGTLVTVSS (SEQ ID NO:21) |
| | VL | AIEMTQSPPSLSASVGETVRIRCLASEDIYRGISWYQQKPGKPPTLLIYGASTLQ SGVPPRFSGSGSGTDYTLTIGGVQAEDAATYYCLGGHSYSSAGLTFGAGTKVE IK (SEQ ID NO:22) |

| | | |
|---|---|---|
| (Note: The CDR regions of heavy and light chain variable regions are underlined and determined using the Kabat numbering scheme) | | |

**Table 4. The CDR regions of anti-CD40 rabbit monoclonal antibodies (using the Kabat numbering scheme)**

| No. | Heavy chain CDR | | Light chain CDR | |
|---|---|---|---|---|
| 5A9 | HCDR1 | SYDMS (SEQ ID NO:23) | LCDR1 | QSSEDISSNLS (SEQ ID NO:26) |
| | HCDR2 | AIGGAGGTYYASWAKS (SEQ ID NO:24) | LCDR2 | AASNLAS (SEQ ID NO:27) |
| | HCDR3 | GWTRLDL (SEQ ID NO:25) | LCDR3 | QGGYWSGISNFGNG (SEQ ID NO:28) |
| 9E6 | HCDR1 | SYDMS (SEQ ID NO:23) | LCDR1 | QASEDISSNLA (SEQ ID NO:29) |
| | HCDR2 | AIGGAGGTYYASWAKS (SEQ ID NO:24) | LCDR2 | PASNLAS (SEQ ID NO:30) |
| | HCDR3 | GWTRLDL (SEQ ID NO:25) | LCDR3 | QGGYWTSTSNFGNG (SEQ ID NO:31) |
| 9F10 | HCDR1 | SYDMS (SEQ ID NO:23) | LCDR1 | QASEDISSNLA (SEQ ID NO:29) |
| | HCDR2 | AIGGAGGTYYASWAKS (SEQ ID NO:24) | LCDR2 | AASNLAS (SEQ ID NO:27) |
| | HCDR3 | GWTRLDL (SEQ ID NO:25) | LCDR3 | QGAYWSSTSYFGNG (SEQ ID NO:32) |
| 4F6 | HCDR1 | SYGVT (SEQ ID NO:33) | LCDR1 | QASQSISNGLA (SEQ ID NO: 3 6) |
| | HCDR2 | AIGSTGSAYYASWAKS (SEQ ID NO:34) | LCDR2 | GASNLAS (SEQ ID NO:37) |
| | HCDR3 | GGITAYAI (SEQ ID NO:35) | LCDR3 | QSYYNSFTTV (SEQ ID NO:38) |
| 5B8 | HCDR1 | SYGVS (SEQ ID NO:39) | LCDR1 | QASEDITNGIA (SEQ ID NO:42) |
| | HCDR2 | AIGSSGSAYYASWARS (SEQ ID NO:40) | LCDR2 | GASNLAS (SEQ ID NO:37) |
| | HCDR3 | GGITVYAI (SEQ ID NO:41) | LCDR3 | QSYYSSSYTI (SEQ ID NO:43) |
| 8G10 | HCDR1 | SYGVS (SEQ ID NO:39) | LCDR1 | QASQSITNGLA (SEQ ID NO:45) |
| | HCDR2 | GIASTGTTYYANWAKS (SEQ ID NO:44) | LCDR2 | GASNLAS (SEQ ID NO:37) |
| | HCDR3 | GGITA YAI (SEQ ID NO:35) | LCDR3 | QSYYDSSSTV (SEQ ID NO:46) |
| 8C12 | HCDR1 | SYGVS (SEQ ID NO:39) | LCDR1 | QASQSISNGLA (SEQ ID NO:36) |
| | HCDR2 | GIGSDGSAYYASWAKS (SEQ ID NO:47) | LCDR2 | GASNLAS (SEQ ID NO:37) |
| | HCDR3 | GGITVYAI (SEQ ID NO:41) | LCDR3 | QSYFSSSSTV (SEQ ID NO:48) |
| 4D4 | HCDR1 | SYGVS (SEQ ID NO:39) | LCDR1 | QASQSISNGLA (SEQ ID NO:36) |
| | HCDR2 | GIGSDGSAYYASWAKS (SEQ ID NO:47) | LCDR2 | GASNLAS (SEQ ID NO:37) |
| | HCDR3 | GGITVYAI (SEQ ID NO:41) | LCDR3 | QSYFSSSSTV (SEQ ID NO:48) |
| 2F12 | HCDR1 | SYGVS (SEQ ID NO:39) | LCDR1 | QASQSISNGLA (SEQ ID NO:36) |
| | HCDR2 | GIGSDGSAYYASWAKS (SEQ ID NO:47) | LCDR2 | GASNLAS (SEQ ID NO:50) |
| | HCDR3 | GGITVYAM (SEQ ID NO:49) | LCDR3 | QSYFSSSSTV (SEQ ID NO:48) |
| 3F6 | HCDR1 | SYAIS (SEQ ID NO:51) | LCDR1 | QSSQSVANNDFLS (SEQ ID NO:54) |
| | HCDR2 | AIDRYGTTYYATWAKS (SEQ ID NO:52) | LCDR2 | GASTLAS (SEQ ID NO:55) |
| | HCDR3 | GPWYYGGDVAWTGSFDP (SEQ ID NO:53) | LCDR3 | TGGYAGPIYI (SEQ ID NO:56) |
| 10A4 | HCDR1 | RNAIS (SEQ ID NO:57) | LCDR1 | LASEDIYRGIS (SEQ ID NO:60) |
| | HCDR2 | GIGSSGSAYYASWAKS (SEQ ID NO:58) | LCDR2 | GASTLQS (SEQ ID NO:61) |
| | HCDR3 | DGYAGSSWGIYYGMDP (SEQ ID NO:59) | LCDR3 | LGGHSYSSAGLT (SEQ ID NO:62) |

The obtained variable region sequences were connected to a human antibody IgG1 constant region (with the mutation N297A, according to the Eu numbering scheme) sequence and a human kappa chain constant region sequence respectively to obtain human-rabbit chimeric antibody sequences. The sequences of the chimeric antibodies were inserted into expression vectors by molecular cloning, and human-rabbit chimeric antibodies were obtained using a HEK293 cell expression system.

### Example 3. Binding of Human-Rabbit Chimeric Anti-CD40 Antibodies to Raji Cells

Raji cells are of a tumor cell line that overexpresses human CD40. 2E5 Raji cells were inoculated onto a 96-well plate. 100 µL of a test antibody was added, with the highest final concentration of 100 nM. The antibody was 5-fold diluted to 8 concentrations. The plate was incubated at 4 °C for 1 h. The plate was washed once with wash buffer, and an AF647-conjugated anti-human IgG antibody (Jackson Immunoresearch Laboratories, Cat. # 205-609-088) was added at a dilution ratio of 1:500. The plate was incubated at 4 °C for 30 min. The plate was washed once with wash buffer, and fluorescence intensity was measured using a flow cytometer. The binding EC₅₀ values of the anti-CD40 antibodies for CD40 were calculated. The anti-CD40 antagonistic antibody CFZ533 (i.e., iscalimab, Nova) was used as a positive control, and the heavy and light chain variable region sequences were derived from sequence 5 and sequence 2 of US8277810B, respectively.

The results in Table 5 show that 2F12, 3F6, 4F6, 5A9, 5B8, 9E6 and 10A4 all have stronger binding EC₅₀ than CFZ533, while other antibodies (taking 8E1 as an example, sequences not shown) have weaker EC₅₀ than CFZ533.

**Table 5. The FACS binding EC₅₀ (nM) of human-rabbit chimeric anti-CD40 antibodies for Raji cells**

| No. | Binding EC₅₀ (nM) |
|---|---|
| 2F12 | 0.412 |
| 3F6 | 1.506 |
| 4F6 | 0.258 |
| 5A9 | 0.671 |
| 5B8 | 0.435 |
| 8E1 | 2.511 |
| 9E6 | 0.608 |
| 10A4 | 0.467 |
| CFZ533 | 2.410 |

### Example 4. Reporter Gene Cell Activity of Human-Rabbit Chimeric Anti-CD40 Antibodies

HEK-Blue CD40L cells were purchased from Invivogen (Cat. # hkb-cd40). The cells were stably transfected with the human CD40 gene and the NF-kB-mediated SEAP genome. The activation level of the CD40 signaling pathway can be characterized by detecting the secreted SEAP in the supernatant using QUANTI-Blue, the substrate of SEAP. In this experiment, the *in vitro* antagonist activity of anti-CD40 antibodies was assessed according to IC₅₀ by detecting the activation of HEK-Blue CD40L cells by CD40L. HEK-Blue CD40L cells were cultured in a DMEM medium containing 10% FBS, 100 µg/mL Zeocin and 30 µg/mL Blasticidin and passaged 2-3 times in a week at a ratio of 1:5 or 1:10. During passaging, the medium was pipetted off, and the cell layer was rinsed with 5 mL of 0.25% pancreatin. Then the pancreatin was pipetted off, the cells were digested in an incubator for 3-5 min, and then a fresh medium was added to resuspend cells. 100 µL of cell suspension was added to a 96-well cell culture plate at a density of 5 × 10^5 cells/mL. The medium was DMEM containing 10% FBS, 100 µg/mL Zeocin and 30 µg/mL Blasticidin. Only 100 µL of sterile water was added to the periphery of the 96-well plate. The plate was incubated in an incubator for 24 h (37 °C, 5% CO₂). After cell adhesion, serially diluted test antibodies were added at 100 µL/well, and the plate was incubated at 37 °C for 30 min. 25 ng/mL CD40L (R&D, Cat. # 2706-CL) and 2 µg/mL anti-His antibody (R&D, Cat. # MAB050) were added, and the plate was incubated in an incubator for 20-24 h (37 °C, 5% CO2). 20 µL of cell supernatant was taken from each well to a new 96-well flat-bottomed plate, and 180 µL of QUANTI-Blue substrate solution was added. The plate was incubated in the dark in an incubator for 1-3 h. The absorbance at 630 nm was measured with a microplate reader (Thermo MultiSkanFc), and IC₅₀ values were calculated and used to assess the *in vitro* cell activity of the anti-CD40 antibodies. See Table 6. The results show that 2F12, 3F6, 4D4, 4F6, 5A9, 5B8, 8C12, 8G10, 9E6, 9F10 and 10A4 all have similar or slightly stronger reporter cell inhibitory activity IC₅₀ than CFZ533, while other antibodies (taking 2F1 as an example, sequences not shown) have weaker IC₅₀ than CFZ533.

**Table 6. The reporter gene cell activity IC₅₀ of anti-CD40 antibodies (nM)**

| No. | IC₅₀ (nM) |
|---|---|
| 2F12 | 0.1717 |
| 3F6 | 0.3778 |
| 4D4 | 0.197 |
| 4F6 | 0.1823 |
| 5A9 | 0.3173 |
| 5B8 | 0.2033 |
| 8C12 | 0.1729 |
| 8G10 | 0.1556 |
| 9E6 | 0.2206 |
| 9F10 | 0.2171 |
| 10A4 | 0.2172 |
| 2F1 | 0.6849 |
| CFZ533 | 0.2715 |

### Example 5. Humanization of Anti-CD40 Antibodies

The sequences of the heavy and light chain variable regions of the rabbit antibodies 2F12, 3F6, 9E6 and 10A4 were compared with the GermLine database of antibodies to obtain human germline templates with high homology. Human germline templates for the humanization of final antibodies are shown in Table 7.

**Table 7. The human germline templates for the humanization of antibodies**

| No. | Heavy chain variable region | | Light chain variable region | |
|---|---|---|---|---|
| | FR1-FR3 | FR4 | FR1-FR3 | FR4 |
| 2F12 | IGHV2-26*01 | IGHJ1*01 | IGkV1-13*02 | IGKJ4*01 |
| 3F6 | IGHV4-30-4*02 | | IGkV1-13*02 | |
| 9E6 | IGHV4-30-4*02 | | IGkV1-9*01 | |
| 10A4 | IGHV4-4*08 | | IGkV1-6*01 | |

The CDR regions of the rabbit antibodies were grafted to the selected human germline templates to replace the human germline variable regions and recombined with the corresponding human IgG constant regions (preferably, the heavy chain was IgG1 with the mutation N297A and the light chain was κ). Then on the basis of the three-dimensional structures of the rabbit antibodies, back mutation was performed on the embedded residues, the residues that directly interact with the CDR regions, and the residues that greatly affect the conformations of VL and VH, and mutation was performed on potential post-translational modification risk sites to obtain the final humanized molecules. By way of example, the humanized light and heavy chain variable region sequences corresponding to 2F12 and 9E6 are shown (see Table 8 and Table 9), where L represents the light chain, H represents the heavy chain, and the numbers following L and H represent different versions of the humanized sequences containing different back mutations.

**Table 8. The heavy and light chain variable region sequences of the humanized 9E6**

| Heavy and light chain variable region sequences | | Sequence No. |
|---|---|---|
| 9E6-L1 | DIQLTQSPSFLSASVGDRVTITCQASEDISSNLAWYQQKPGKPPK LLIFPASNLASGVPSRFSGSGSGTEFTLTISSLQPEDFATYYCQGG YWTSTSNFGNVFGGGTKVEIK | SEQ ID NO:63 |
| 9E6-L2 | DIQLTQSPSFLSASVGDRVTITCQASEDISSNLAWYQQKPGKPPK LLIFPASNLASGVPSRFSGSGSGTEFTLTISSLQPEDFATYYCQGG YWTSTSNFGSGFGGGTKVEIK | SEQ ID NO:64 |
| 9E6-L3 | DIQLTQSPSFLSASVGDRVTITCQASEDISSNLAWYQQKPGKPPK LLIFPASNLASGVPSRFSGSGSGTEFTLTISSLQPEDFATYYCQGG YWTSTSNFGTGFGGGTKVEIK | SEQ ID NO:65 |
| 9E6-L4 | DIQLTQSPSFLSASVGDRVTITCQASEDISSNLAWYQQKPGKPPK LLIFPASNLASGVPSRFSGSGSGTEFTLTISSLQPEDFATYYCQGG YWTSTSNFGQGFGGGTKVEIK | SEQ ID NO:66 |
| 9E6-H1 | EVQLQESGPGLVKPSDTLSLTCTVSGFSLSSYDMSWIRQPPGKG LEWIGAIGGAGGTYYASWAKSRVTISVDTSLNQVSLKLSSVTAA DTAVYYCARGWTRLDLWGQGTLVTVSS | SEQ ID NO:67 |
| 9E6-H2 | QVQVQESGPGLVKPSDTLSLTCTVSGFSLSSYDMSWIRQPPGKG LEWIGAIGGAGGTYYASWAKSRVTISVDTSLNQVSLKLSSVTAA DTAVYYCARGWTRLDLWGQGTLVTVSS | SEQ ID NO:68 |

(The CDRs defined by the Kabat numbering scheme are underlined.)

During the humanization of 9E6, the following LCDR3s were obtained:
>LCDR3 of 9E6-L1
   QGGYWTSTSNFGNV (SEQ ID NO: 69)
>LCDR3 of 9E6-L2
   QGGYWTSTSNFGSG (SEQ ID NO: 70)
>LCDR3 of 9E6-L3
   QGGYWTSTSNFGTG (SEQ ID NO: 71)
>LCDR3 of 9E6-L4
   QGGYWTSTSNFGQG (SEQ ID NO: 72)
>General formula for LCDR3s of 9E6-Ls
   QGGYWTSTSNFGX₉X₁₀ (SEQ ID NO:73), wherein X₉ is selected from the group consisting of N, S, T and Q, and X₁₀ is selected from the group consisting of V and G. 5A9, 9E6 and 9F10 have the following general formula structure:
   the HCDR1, HCDR2 and HCDR3 are represented by SYDMS (SEQ ID NO:23), AIGGAGGTYYASWAKS (SEQ ID NO: 24) and GWTRLDL (SEQ ID NO: 25), respectively;
   the LCDR1 is represented by QX₁SEDISSNLX₂ (SEQ ID NO: 74), wherein X₁ is selected from the group consisting of A and S, and X₂ is selected from the group consisting of A and S;
   the LCDR2 is represented by X₃ASNLAS (SEQ ID NO: 75), wherein X₃ is selected from the group consisting of A and P;
   the LCDR3 is represented by QGX₄YWX₅X₆X₇SX₈FGX₉X₁₀ (SEQ ID NO: 76), wherein X₄ is selected from the group consisting of A and G, X₅ is selected from the group consisting of S and T, X₆ is selected from the group consisting of S and G, X₇ is selected from the group consisting of T and S, X₈ is selected from the group consisting of N and Y, X₉ is selected from the group consisting of N, S, T and Q, and X₁₀ is selected from the group consisting of V and G.

**Table 9. The heavy and light chain variable region sequences of the humanized 2F12**

| | Heavy and light chain variable region sequences | Sequence No. |
|---|---|---|
| 2F12-L1 | AFQLTQSPSSLSASVGDRVTITCQASQSISNVLAWYQQKPGKPP KLLIVGASNLASGVPSRFSGSGSGTDFTLTISSLQPEDFATYYCQ SYFSSSSTVFGGGTKVEIK | SEQ ID NO:77 |
| 2F12-L2 | AFQLTQSPSSLSASVGDRVTITCQASQSISQGLAWYQQKPGKPP KLLIVGASNLASGVPSRFSGSGSGTDFTLTISSLQPEDFATYYCQ SYFSSSSTVFGGGTKVEIK | SEQ ID NO:78 |
| 2F12-L3 | AFQLTQSPSSLSASVGDRVTITCQASQSISSGLAWYQQKPGKPP KLLIVGASNLASGVPSRFSGSGSGTDFTLTISSLQPEDFATYYCQ SYFSSSSTVFGGGTKVEIK | SEQ ID NO:79 |
| 2F12-L4 | AFQLTQSPSSLSASVGDRVTITCQASQSISTGLAWYQQKPGKPP KLLIVGASNLASGVPSRFSGSGSGTDFTLTISSLQPEDFATYYCQ SYFSSSSTVFGGGTKVEIK | SEQ ID NO:80 |
| 2F12-H1 | EVTLKESGPVLVKPTETLTLTCTVSGFSLSSYGVSWIRQPPGKAL EWLAGIGSDGSAYYASWAKSRLTISRDTNLKQVVLTMTNMDPV DTATYYCARGGITVYAMWGQGTLVTVSS | SEQ ID NO:81 |
| 2F12-H2 | EVTVKESGPVLVKPTETLTLTCTVSGFSLSSYGVSWIRQPPGKA LEWLGGIGSDGSAYYASWAKSRLTISRDTNLKQVVLTMTNMDP VDTATYYCARGGITVYAMWGQGTLVTVSS | SEQ ID NO:82 |

(The CDRs defined by the Kabat numbering scheme are underlined.)

During the humanization of 2F12, the following LCDR1s were obtained:
>LCDR1 of2F12-L1
   QASQSISNVLA (SEQ ID NO: 83)
>LCDR1 of2F12-L2
   QASQSISQGLA (SEQ ID NO: 84)
>LCDR1 of2F12-L3
   QASQSISSGLA (SEQ ID NO: 85)
>LCDR1 of 2F12-L4
   QASQSISTGLA (SEQ ID NO: 86)
>General formula for LCDR1s of 2F12-Ls
   QASQSISX₂₄X₂₅LA (SEQ ID NO: 87), wherein X₂₄ is selected from the group consisting of N, Q, S and T, and X₂₅ is selected from the group consisting of V and G. 4F6, 5B8, 8G10, 8C12, 4D4 and 2F12 have the following general formula structure:
   the HCDR1 is represented by SYGVX₁₁ (SEQ ID NO: 88), wherein X₁₁ is selected from the group consisting of S and T;
   the HCDR2 is represented by X₁₂IX₁₃SX₁₄GX₁₅X₁₆YYAX₁₇WAX₁₈S (SEQ ID NO: 89), wherein X₁₂ is selected from the group consisting of A and G, X₁₃ is selected from the group consisting of G and A, X₁₄ is selected from the group consisting of T, S and D, X₁₅ is selected from the group consisting of T and S, X₁₆ is selected from the group consisting of T and A, X₁₇ is selected from the group consisting of S and N, and X₁₈ is selected from the group consisting of K and R;
   the HCDR3 is represented by GGITX₁₉YAX₂₀ (SEQ ID NO: 90), wherein X₁₉ is selected from the group consisting of A and V, and X₂₀ is selected from the group consisting of I and M;
   the LCDR1 is represented by QASX₂₁X₂₂IX₂₃X₂₄X₂₅LA (SEQ ID NO: 91), wherein X₂₁ is selected from the group consisting of Q and E, X₂₂ is selected from the group consisting of S and D, X₂₃ is selected from the group consisting of S and T, X₂₄ is selected from the group consisting of N, Q, S and T, and X₂₅ is selected from the group consisting of V and G.
   the LCDR2 is represented by GASNLAS (SEQ ID NO: 37);
   the LCDR3 is represented by QSYX₂₆X₂₇SX₂₈X₂₉TX₃₀ (SEQ ID NO: 92), wherein X₂₆ is selected from the group consisting of F and Y, X₂₇ is selected from the group consisting of S, D and N, X₂₈ is selected from the group consisting of S and F, X₂₉ is selected from the group consisting of S, T and Y, and X₃₀ is selected from the group consisting of V and I.

The various versions of light and heavy chains of each of the rabbit antibodies were combined in pairs, expressed and purified. The names are combinations of the heavy chain variable region number and the light chain variable region number. For example, "2F12-L1H1" refers to an antibody with SEQ ID NO: 63 as the VH and SEQ ID NO: 67 as the VL. The light and heavy chain constant regions used the human IgG1 constant region (with the mutation N297A, according to the Eu numbering scheme) sequence and the human kappa chain constant region sequence, respectively.

By way of example, the sequences of the humanized molecules 2F12-L4H2 (using the 2F12-L4 light chain and the 2F12-H2 heavy chain) and 9E6-L4H2 (using the 9E6-L4 light chain and the 9E6-H2 heavy chain) are shown below, where the light and heavy chain constant regions used the human IgG1 constant region (with the mutation N297A, according to the Eu numbering scheme) sequence and the human kappa chain constant region sequence, respectively.
>9E6-L4H2 HC: 
QVQVQESGPGLVKPSDTLSLTCTVSGFSLSSYDMSWIRQPPGKGLEWIGAIGGA
GGTYYASWAKSRVTISVDTSLNQVSLKLSSVTAADTAVYYCARGWTRLDLWGQ
GTLVTVSSASTKGPSVFPLAPSSKSTSGGTAALGCLVKDYFPEPVTVSWNSGALT
SGVHTFPAVLQSSGLYSLSSVVTVPSSSLGTQTYICNVNHKPSNTKVDKKVEPKS
CDKTHTCPPCPAPELLGGPSVFLFPPKPKDTLMISRTPEVTCVVVDVSHEDPEVK
FNWYVDGVEVHNAKTKPREEQYASTYRVVSVLTVLHQDWLNGKEYKCKVSN
KALPAPIEKTISKAKGQPREPQVYTLPPSREEMTKNQVSLTCLVKGFYPSDIAVE
WESNGQPENNYKTTPPVLDSDGSFFLYSKLTVDKSRWQQGNVFSCSVMHEALH
NHYTQKSLSLSPGK
SEQ ID NO: 93
>9E6-L4H2 LC: 
DIQLTQSPSFLSASVGDRVTITCQASEDISSNLAWYQQKPGKPPKLLIFPASNLAS
GVPSRFSGSGSGTEFTLTISSLQPEDFATYYCQGGYWTSTSNFGQGFGGGTKVEI
KRTVAAPSVFIFPPSDEQLKSGTASVVCLLNNFYPREAKVQWKVDNALQSGNSQ
ESVTEQDSKDSTYSLSSTLTLSKADYEKHKVYACEVTHQGLSSPVTKSFNRGEC
SEQ ID NO: 94
>2F12-L4H2 HC: 
EVTVKESGPVLVKPTETLTLTCTVSGFSLSSYGVSWIRQPPGKALEWLGGIGSDG
SAYYASWAKSRLTISRDTNLKQVVLTMTNMDPVDTATYYCARGGITVYAMWG
QGTLVTVSSASTKGPSVFPLAPSSKSTSGGTAALGCLVKDYFPEPVTVSWNSGA
LTSGVHTFPAVLQSSGLYSLSSVVTVPSSSLGTQTYICNVNHKPSNTKVDKKVEP
KSCDKTHTCPPCPAPELLGGPSVFLFPPKPKDTLMISRTPEVTCVVVDVSHEDPE
VKFNWYVDGVEVHNAKTKPREEQYASTYRVVSVLTVLHQDWLNGKEYKCKV
SNKALPAPIEKTISKAKGQPREPQVYTLPPSREEMTKNQVSLTCLVKGFYPSDIAV
EWESNGQPENNYKTTPPVLDSDGSFFLYSKLTVDKSRWQQGNVFSCSVMHEAL
HNHYTQKSLSLSPGK
SEQ ID NO: 95
>2F12-L4H2 LC:

AFQLTQSPSSLSASVGDRVTITCQASQSISTGLAWYQQKPGKPPKLLIVGASNLA
SGVPSRFSGSGSGTDFTLTISSLQPEDFATYYCQSYFSSSSTVFGGGTKVEIKRTVA
APSVFIFPPSDEQLKSGTASVVCLLNNFYPREAKVQWKVDNALQSGNSQESVTE
QDSKDSTYSLSSTLTLSKADYEKHKVYACEVTHQGLSSPVTKSFNRGEC
SEQ ID NO: 96

The heavy chain constant regions of the above antibodies were engineered. The antibodies used the human IgG1 constant region and contained 5 point mutations: L234A, L235A, M252Y, S254T and T256E (according to the Eu numbering scheme), and the light chain constant region and the light and heavy chain variable regions were unchanged. The newly produced antibodies were 2F12-L4H2-AAYTE and 9E6-L4H2-AAYTE, the complete heavy chain sequences of which are shown below:
>9E6-L4H2-AAYTE HC: 
QVQVQESGPGLVKPSDTLSLTCTVSGFSLSSYDMSWIRQPPGKGLEWIGAIGGA
GGTYYASWAKSRVTISVDTSLNQVSLKLSSVTAADTAVYYCARGWTRLDLWGQ
GTLVTVSSASTKGPSVFPLAPSSKSTSGGTAALGCLVKDYFPEPVTVSWNSGALT
SGVHTFPAVLQSSGLYSLSSVVTVPSSSLGTQTYICNVNHKPSNTKVDKKVEPKS
CDKTHTCPPCPAPEAAGGPSVFLFPPKPKDTLYITREPEVTCVVVDVSHEDPEVK
FNWYVDGVEVHNAKTKPREEQYNSTYRVVSVLTVLHQDWLNGKEYKCKVSN
KALPAPIEKTISKAKGQPREPQVYTLPPSREEMTKNQVSLTCLVKGFYPSDIAVE
WESNGQPENNYKTTPPVLDSDGSFFLYSKLTVDKSRWQQGNVFSCSVMHEALH
NHYTQKSLSLSPGK
SEQ ID NO: 97
>2F12-L4H2-AAYTE HC:

EVTVKESGPVLVKPTETLTLTCTVSGFSLSSYGVSWIRQPPGKALEWLGGIGSDG
SAYYASWAKSRLTISRDTNLKQVVLTMTNMDPVDTATYYCARGGITVYAMWG
QGTLVTVSSASTKGPSVFPLAPSSKSTSGGTAALGCLVKDYFPEPVTVSWNSGA

LTSGVHTFPAVLQSSGLYSLSSVVTVPSSSLGTQTYICNVNHKPSNTKVDKKVEP
KSCDKTHTCPPCPAPEAAGGPSVFLFPPKPKDTLYITREPEVTCVVVDVSHEDPE
VKFNWYVDGVEVHNAKTKPREEQYNSTYRVVSVLTVLHQDWLNGKEYKCKV
SNKALPAPIEKTISKAKGQPREPQVYTLPPSREEMTKNQVSLTCLVKGFYPSDIAV
EWESNGQPENNYKTTPPVLDSDGSFFLYSKLTVDKSRWQQGNVFSCSVMHEAL
HNHYTQKSLSLSPGK
SEQ ID NO: 98

The heavy and light chain constant regions are underlined.

Exemplary IgG Fes are shown below:
> IgG1-Fc (N297A) 
ASTKGPSVFPLAPSSKSTSGGTAALGCLVKDYFPEPVTVSWNSGALTSGVHTFPA
VLQSSGLYSLSSVVTVPSSSLGTQTYICNVNHKPSNTKVDKKVEPKSCDKTHTC
PPCPAPELLGGPSVFLFPPKPKDTLMISRTPEVTCVVVDVSHEDPEVKFNWYVD
GVEVHNAKTKPREEQYASTYRVVSVLTVLHQDWLNGKEYKCKVSNKALPAPIE
KTISKAKGQPREPQVYTLPPSREEMTKNQVSLTCLVKGFYPSDIAVEWESNGQPE
NNYKTTPPVLDSDGSFFLYSKLTVDKSRWQQGNVFSCSVMHEALHNHYTQKSL
SLSPGK
SEQ ID NO: 99
> IgG1-Fc (AAYTE)

ASTKGPSVFPLAPSSKSTSGGTAALGCLVKDYFPEPVTVSWNSGALTSGVHTFPA
VLQSSGLYSLSSVVTVPSSSLGTQTYICNVNHKPSNTKVDKKVEPKSCDKTHTC
PPCPAPEAAGGPSVFLFPPKPKDTLYITREPEVTCVVVDVSHEDPEVKFNWYVD
GVEVHNAKTKPREEQYNSTYRVVSVLTVLHQDWLNGKEYKCKVSNKALPAPIE
KTISKAKGQPREPQVYTLPPSREEMTKNQVSLTCLVKGFYPSDIAVEWESNGQPE
NNYKTTPPVLDSDGSFFLYSKLTVDKSRWQQGNVFSCSVMHEALHNHYTQKSL
SLSPGK
SEQ ID NO: 100

### Example 6. Binding of Humanized Anti-CD40 Antibodies to Raji Cells

Raji cells highly express human CD40 on the cell surface and thus can be used to detect the binding of CD40 antagonistic antibodies to the cell surface human CD40.

Raji cells were plated at 1.5E5/well, and various concentrations of CD40 antagonistic antibodies were added. The plate was incubated at 4 °C for 1 h. After two washes with FACS buffer (PBS + 2% FBS), a secondary antibody (Alexa Flour488 conjugated anti-human IgG (H+L) antibody) was added, and the plate was incubated at 4 °C for 0.5 h. After two washes with FACS buffer, the fluorescence intensity on the cell surface was measured by flow cytometry (BD FACS Celesta).

According to the results shown in Table 10, 2F12-L4H2, 9E6-L4H2 and CFZ533 have similar abilities to bind to Raji cell surface human CD40.

**Table 10. The EC₅₀ results for the FACS binding of humanized anti-CD40 antibodies to Raji cells**

| Antibody No. | Binding EC₅₀ (nM) | Antibody No. | Binding EC₅₀ (nM) |
|---|---|---|---|
| 2F12 | 0.479 | 91:6 | 0.895 |
| 2F12-L1H1 | 0.549 | 9E6-L1H1 | 1.083 |
| 2F12-L1H2 | 0.469 | 9E6-L1H2 | 1.048 |
| 2F12-L2H1 | 0.516 | 9E6-L2H1 | 1.993 |
| 2F12-L2H2 | 0.516 | 9E6-L2H2 | 1.660 |
| 2F12-L3H1 | 0.456 | 9E6-L3H1 | 1.065 |
| 2F12-L3H2 | 0.762 | 9E6-L3H2 | 1.148 |
| 2F12-L4H1 | 0.508 | 9E6-L4H1 | 0.928 |
| 2F12-L4H2 | 0.589 | 9E6-L4H2 | 0.870 |
| CFZ533 | 1.009 | | |

### Example 7. Assays for Affinities of Anti-CD40 Antibodies for Human CD40 and Cynomolgus Monkey CD40

The test antibody was affinity-captured on an anti-human Fc chip, and a series of concentrations of His-tagged human or cynomolgus monkey CD40 antigen were allowed to flow over the chip surface. Reaction signals were detected in real time using a Biacore instrument, and thus binding and dissociation curves were obtained. The buffer used in the experiment was HBS-EP + 10× buffer solution (Cat. # BR-1006-69, GE) diluted to 1× (pH 7.4) with D. I. Water. Fitting was performed on the data obtained from the experiment using a (1:1) Binding model to obtain affinity values. See Table 11.

9E6-L4H2 and 2F12-L4H2 have similar binding constants for human CD40 to CFZ533, 9E6-L4H2 has a stronger binding affinity for cynomolgus monkey CD40, and 2F12-L4H2 has a slightly weak binding affinity for cynomolgus monkey CD40.

**Table 11. The SPR affinities of CD40 antagonistic antibodies for CD40 of different species**

| Mobile phase | Stationary phase | Ka (1/Ms) | Kd (1/s) | KD (M) |
|---|---|---|---|---|
| Human CD40 | 2F12-L4H2 | 7.58E+05 | 2.06E-03 | 2.71E-09 |
| | 9E6-L4H2 | 4.13E+05 | 2.21E-04 | 5.36E-10 |
| | CFZ533 | 6.88E+05 | 1.41E-04 | 2.05E-10 |
| Cynomolgus monkey CD40 | 2F12-L4H2 | 1.17E+07 | 4.14E-01 | 3.53E-08 |
| | 9E6-L4H2 | 5.14E+05 | 6.23E-05 | 1.21E-10 |

### Example 8: Reporter Gene Cell Activity of Anti-CD40 Antibodies

HEK-Blue CD40L cells were purchased from Invivogen (Cat. # hkb-cd40). The cells were stably transfected with the human CD40 gene and the NF-kB-mediated SEAP genome. The activation level of the CD40 signaling pathway can be characterized by measuring the amount of secreted SEAP in the supernatant using QUANTI-Blue, the substrate of SEAP. In the assays, the inhibitory effects of the CD40 antagonistic antibodies on the CD40L-induced HEK-Blue CD40L cell activation were measured, and the *in vitro* cell activity of the CD40 antagonistic antibodies was assessed based on IC₅₀. HEK-Blue CD40L cells were cultured in a DMEM medium containing 10% FBS, 100 µg/mL Normocin, 100 µg/mL Zeocin and 30 pg/mL Blasticidin, with 2-3 passages a week. HEK-Blue CD40L cells were plated at 5E4/well into a 96-well cell culture plate (the medium was DMEM, 10% FBS, 100 µg/mL Normocin) and incubated overnight. After cell adhesion, serially diluted test antibodies were added at 100 µL/well, and the plate was incubated at 37 °C for 1 h. CD40L-his (R&D, 2706-CL-025) and anti-His antibody (R&D, MAB050-500) were added, and the plate was incubated overnight. After cell centrifugation, 20 µL of the cell supernatant was transferred to a new 96-well white plate, and 180 µL of QUANTI-Blue substrate solution was added. The plate was incubated in the dark for 15 min. The absorbance at 620 nm was measured with an Envision microplate reader, and IC₅₀ values were calculated and used to assess the *in vitro* cell activity of the CD40 antagonistic antibodies.

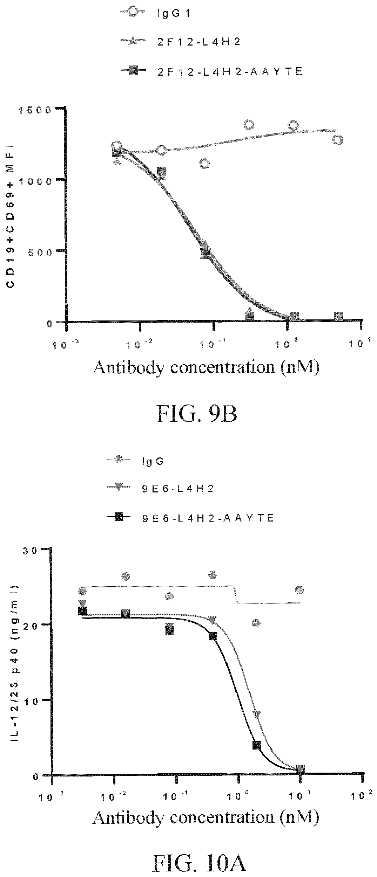

According to the results shown in Table 12 and FIGs. 1A to 1B, 9E6-L4H2 and CFZ533 have similar inhibitory activity against the reporter gene system, and 2F12-L4H2 has better inhibitory activity than CFZ533.

**Table 12. The IC₅₀ for the inhibition of CD40 reporter gene cell activity by anti-CD40 antibodies**

| | Antibody No. | IC₅₀ (nM) |
|---|---|---|
| Assay 1 | 2F12-L4H2 | 0.18 |
| | CFZ533 | 0.36 |
| Assay 2 | 9E6-L4H2 | 0.50 |
| | CFZ533 | 0.35 |

### Example 9. Inhibitory Activity of Anti-CD40 Antibodies in B Cell Activation Assay System

CD40 is highly expressed in B cells, and CD40L, upon binding to CD40, can induce B cell activation and up-regulate the expression of a series of activation markers. CD40 antagonistic antibodies block the binding of CD40L to CD40, thereby eliminating the immune activation process of B cells.

Human PBMCs were plated at 2E5/well, 50 µL/well into a 96-well cell culture plate (the medium was RPMI-1640, 10% FBS, 1% penicillin-streptomycin). Serially diluted test antibodies were added at 50 µL/well, and the cells were co-incubated at 37 °C in 5% CO₂ for 0.5 h. CD40L-his and an anti-His antibody were added to each well for overnight stimulation. The following day, the plate was centrifuged, the supernatant was removed, and the cells were washed twice with FACS buffer and stained at room temperature for 15 min with 100 µL of Fixable viability dye EF780 (Invitrogen, 65086514) diluted at 1: 1000. The cells were washed twice and then blocked at room temperature for 10 min with 100 µL of human Fc blocker (BD, 564220) diluted at 1:200. After centrifugation, the cells were incubated at 4 °C for 0.5 h with flow cytometry antibodies (PerCP/Cyanine5.5 anti-human CD19 (Biolegend, 302230), and APC anti-human CD69 (Biolegend, 310910)) diluted at 1:200. After centrifugation, the supernatant was removed, the cells were washed twice with FACS buffer and then resuspended in 200 µL of PBS, and the fluorescence intensity on the cell surface was measured by flow cytometry (BD FACS Celesta).

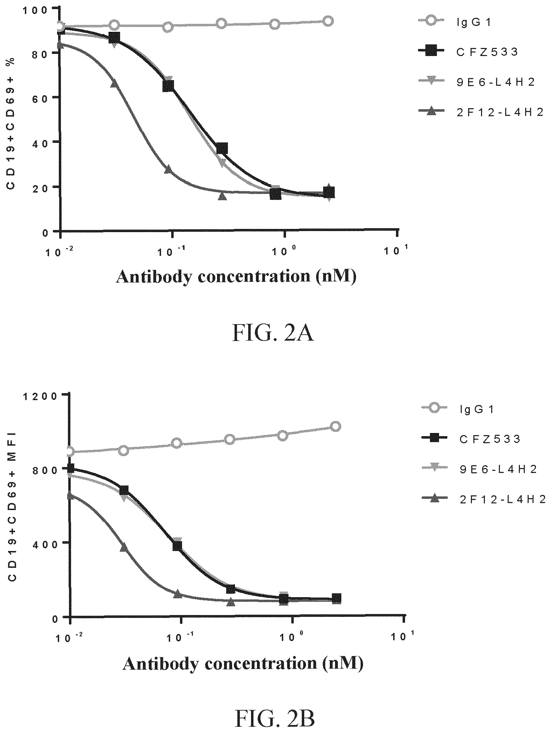

According to the results shown in Table 13 and FIGs. 2A to 2B, 9E6-L4H2 has similar B cell activation inhibition activity to CFZ533. 2F12-L4H2 has greater B cell inhibition activity than CFZ533.

**Table 13. The inhibitory activity of humanized anti-CD40 antibodies in the B cell activation assay system**

| | CD19+CD69+% | | CD19+CD69+ MFI | |
|---|---|---|---|---|
| Antibody No. | IC₅₀ (nM) | Maximum inhibition rate | IC₅₀ (nM) | Maximum inhibition rate |
| CFZ533 | 0.14 | 95% | 0.07 | 110% |
| 9E6-L4H2 | 0.14 | 98% | 0.07 | 110% |
| 2F12-L4H2 | 0.04 | 93% | 0.03 | 111% |

After antibody addition, the CD19+CD69+ signal was lower than the background, probably because the background activation of B cells was inhibited to some extent (in addition to the inhibition of the CD40L-induced signal).

### Example 10. Inhibitory Activity of Anti-CD40 Antibodies in DC Cell Activation Assay System

CD40 is highly expressed on dendritic cells (DCs), and CD40L, upon binding to CD40, can induce DC activation, up-regulate the expression of multiple activation markers on the DC Cell surface, and promote the secretion of multiple inflammatory factors from DCs, further amplifying immune responses. CD40 antagonistic antibodies block the binding of CD40L to CD40, thereby eliminating the immune activation process of DC cells.

Monocytes were sorted and enriched from fresh primary human peripheral blood PBMCs using the EsaySepTM human CD14 sorting kit (Stemcell, 19359), and differentiated for 6 days with RPMI-1640 medium (10% FBS, 1% penicillin-streptomycin), 50 ng/mL IL-4 (PeproTech, 200-04) and 50 ng/mL GM-CSF (PeproTech, 300-03). On day 7, the differentiated DC cells were plated at 1E5/well into a 96-well cell culture plate. Serially diluted test antibodies were added to the wells, and the cells were co-incubated at 37 °C in 5% CO₂ for 0.5 h. CD40L-his and an anti-His antibody were then added to each well at final concentrations. After 48 h of culture, the level of DC cell activation was measured by flow cytometry: after centrifugation, the supernatant was removed, and the cells were washed twice with FACS buffer (PBS + 2% FBS) and stained at room temperature for 15 min with 100 µL of Fixable viability dye EF780 (Invitrogen, 65086514) diluted at 1: 1000. The cells were washed twice and then blocked at room temperature for 10 min with 100 µL of human Fc blocker (BD, 564220) diluted at 1:200. After centrifugation, the cells were incubated at 4 °C for 0.5 h with flow cytometry antibodies (Alexa Fluor^{®} 700 anti-human CD11c (Biolegend, 337220), Brilliant Violet 421^{™} anti-human CD80 (Biolegend, 305221), and APC anti-human CD86 (Biolegend, 305412)) diluted at 1:200. After centrifugation, the supernatant was removed, the cells were washed twice with FACS buffer and then resuspended in 200 µL of PBS, and the fluorescence intensity on the cell surface was measured by flow cytometry (BD FACS Celesta).

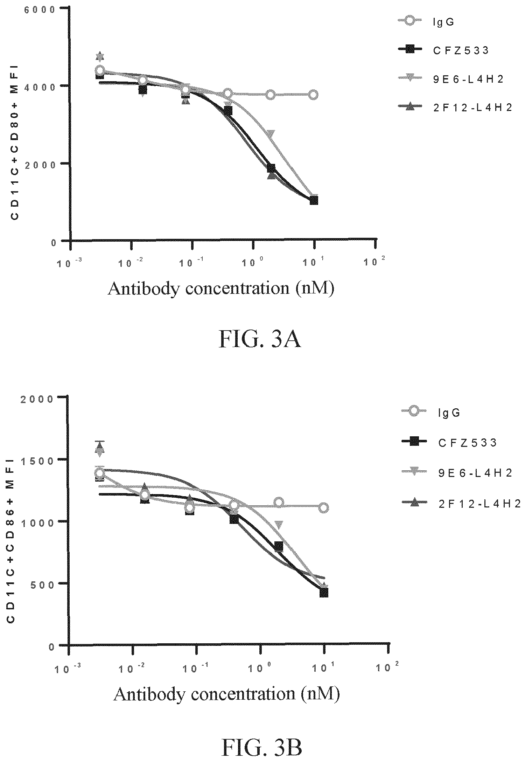

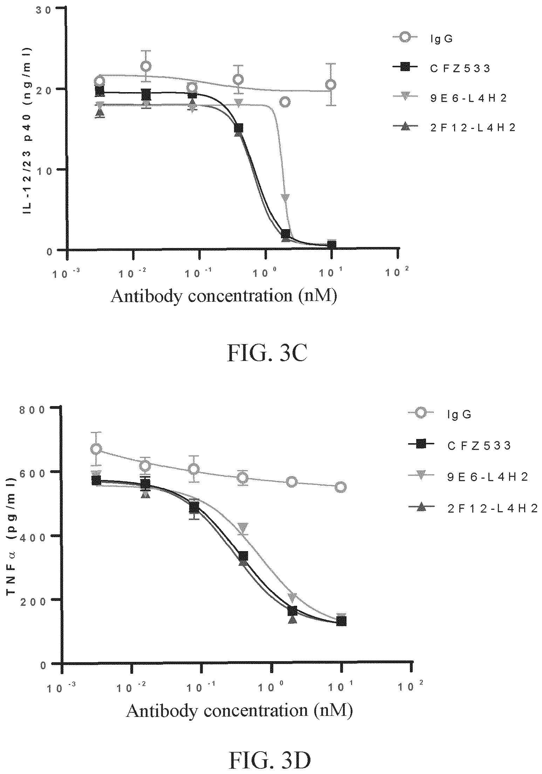

In addition, the level of cytokine secretion in the supernatant was measured after 24 h (TNFα, Cisbio, 62HTNFAPEG) and 48 h (IL-12/23 p40, Novus, VAL121) of culture. According to the results shown in Table 14 and FIGs. 3A to 3D, 9E6-L4H2 and 2F12-L4H2 have similar DC cell inhibition activity to the control antibody CFZ533.

**Table 14. The inhibitory activity of humanized anti-CD40 antibodies in the DC cell activation assay system**

| Antibody No. | IC₅₀ (nM) in DC cell activation assay system | | | |
|---|---|---|---|---|
| | CD80 MFI | CD86 MFI | IL-12/23 p40 | TNFα |
| CFZ533 | 1.19 | 1.93 | 0.68 | 0.35 |
| 9E6-L4H2 | 3.44 | 3.72 | 1.85 | 0.7 |
| 2F12-L4H2 | 0.78 | 0.52 | 0.68 | 0.30 |

### Example 11. Agonistic Activity of Humanized Anti-CD40 Antibodies in B Cell Activation Assay System

CD40, which belongs to the TNF superfamily of receptors, mediates the specific and non-specific activation of downstream signaling pathways upon binding to the ligand CD40L or being cross-linked by antibodies. Therefore, the background agonist activity of CD40 antagonistic antibodies on B cells can be measured without adding CD40L.

Human PBMCs were plated at 2E5/well, 100 µL/well into a 96-well cell culture plate (the medium was RPMI-1640, 10% FBS, 1% penicillin-streptomycin). Serially diluted test antibodies were added at 100 µL/well, and the cells were incubated overnight at 37 °C in 5% CO₂. The following day, the plate was centrifuged and the supernatant was removed, and the cells were washed twice with FACS buffer and stained at room temperature for 15 min with 100 µL of Fixable viability dye EF780 diluted at 1: 1000. The cells were washed twice and then blocked at room temperature for 10 min with 100 µL of human Fc blocker diluted at 1:200. After centrifugation, the cells were incubated at 4 °C for 0.5 h with flow cytometry antibodies (PerCP/Cyanine5.5 anti-human CD19 (Biolegend, 302230), and APC anti-human CD69 (Biolegend, 310910)) diluted at 1:200. After centrifugation, the supernatant was removed, the cells were washed twice with FACS buffer and then resuspended in 200 µL of PBS, and the fluorescence intensity on the cell surface was measured by flow cytometry (BD FACS Celesta).

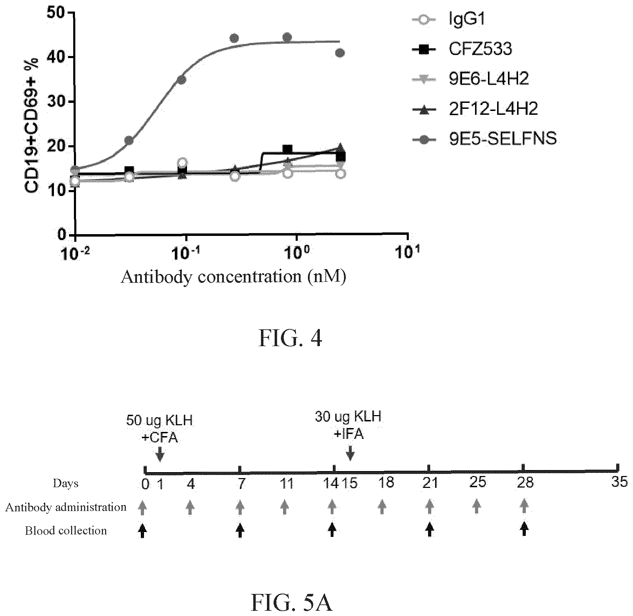

According to the results shown in FIG. 4, the CD40 agonistic antibody 9E5-SELFNS (WO2020108611A1) activated B cells in a dose-dependent manner, while 9E6-L4H2 and 2F12-L4H2 still showed no significant B cell agonistic activity at 2.5 nM.

### Example 12. Mouse T Cell-Dependent Humoral Immune Response (TDAR) Model

Female human CD40 transgenic mice, 6-7 weeks old, were purchased from Biocytogen Jiangsu Co., Ltd. Housing environment: SPF; production license: SCXK(Jiangsu)-2016-0004; human CD40 transgenic mouse certification number: 320726200100167773. After arrival, the animals were acclimatized for 7 days and randomly grouped. On day 0 of the experiment, one of the mice in each group was subjected to blood collection followed by intraperitoneal injection. On day 1, each mouse was intraperitoneally injected with 50 µg of KLH (KLH:complete Freund's adjuvant (CFA) = 1: 1 emulsified immune complex) for immunization. On day 15 of the experiment, each mouse was intraperitoneally injected with 50 µg of KLH (KLH:incomplete Freund's adjuvant (IFA) = 1:1 emulsified immune complex) for a second immunization. Each group was intraperitoneally injected twice a week, and blood samples of about 150 µL were collected from the orbital venous plexus on days 7, 14, 21, and 28. Whole blood was left to stand at room temperature for 1-4 h and centrifuged at 7000 rpm at 4 °C for 10 min to separate serum, and the serum was stored at -80 °C before use. The specific experimental process is shown in FIG. 5A. The specific administration regimens are shown in Table 15. Mouse serum was separated weekly and assayed by ELISA for the anti-KLH specific IgG level.

**Table 15. Experimental administration regimens for the mouse TDAR model**

| | Group | Drug | N | Dose | Frequency |
|---|---|---|---|---|---|
| hCD40 transgenic mouse | 1 | IgG1 | 5 | 1 mg/kg | BIW |
| | 2 | CFZ533 | 5 | 1 mg/kg | BIW |
| | 3 | CFZ533 | 5 | 0.3 mg/kg | BIW |
| | 4 | 9E6-L4H2 | 5 | 1 mg/kg | BIW |
| | 5 | 9E6-L4H2 | 5 | 0.3 mg/kg | BIW |
| | 6 | 2F12-L4H2 | 5 | 1 mg/kg | BIW |
| | 7 | 2F12-L4H2 | 5 | 0.3 mg/kg | BIW |

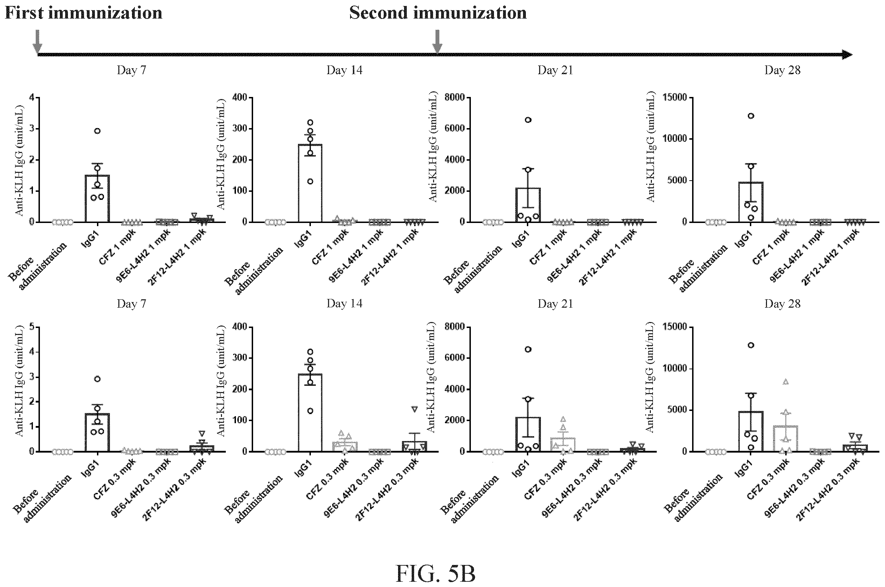

According to the results shown in FIG. 5B and Table 16, 1 mg/kg CD40 antagonistic antibodies significantly inhibited anti-KLH specific IgG production following two immunizations. The low doses (0.3 mg/kg) of 9E6-L4H2 and 2F12-L4H2 better inhibited anti-KLH IgG production than the same dose of CFZ533 following two immunizations.

**Table 16. The inhibition of IgG production by anti-CD40 antibodies after immunization (0.3 mpk)**

| | Day 7 | Day 14 | Day 21 | Day 28 |
|---|---|---|---|---|
| Before administration | 0 | 0 | 0 | 0 |
| IgG1 | 1.5±0.4 | 247.4±33.1 | 2187±1248 | 4773±2280 |
| CFZ533 (0.3 mpk) | 0.04±0.01 | 30.9±10.8 | 845±423 | 3043±1600 |
| 9E6-L4H2 (0.3 mpk) | 0.002±0.001 | 0.02±0.01 | 0 | 18.25±8.9 |
| 2F12-L4H2 (0.3 mpk) | 0.22±0.14 | 33.4±25.8 | 175.5±103 | 805.7±415.6 |

### Example 13. Mouse Skin Graft Rejection Model

Male Balb/c mice, 6 weeks old, were purchased from the Laboratory Animal Management Department, Shanghai Institute of Planned Parenthood Research. Housing environment: SPF; certification number: 20180006023393.

Female human CD40 transgenic mice, 6-7 weeks old, were purchased from Biocytogen Jiangsu Co., Ltd. Housing environment: SPF; certification number: 320726200100179778.

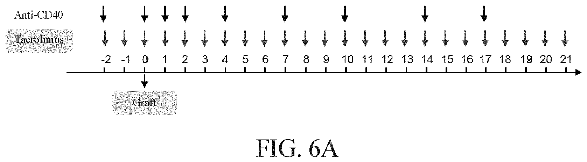

After arrival, the animals were acclimatized for 7 days and randomly grouped. On day -2 of the experiment, the mice were intraperitoneally injected with tacrolimus FK506 or CD40 antagonistic antibodies. On day 0 of the experiment, donor Balb/c mice and C57BL6/J mice were anesthetized with 4% chloral hydrate. The tails of the donor mice were removed, and full circles of skin, 1 cm in length, were isolated from the tails. The hair on the acceptor mice's back was removed, and an incision was made along the skin layer. An equal area of skin was removed while preserving the back fat and connective tissue. The donor skin was placed on the acceptor mice's incision site, and the incision was closed with glue. The mice were then placed back in cages to recover. The specific experimental process is shown in FIG. 6A.

The mice were dosed according to the administration regimens in Table 17. After 7 days of recovery, the mouse skin survival was observed daily and rejection scores were recorded. The scoring system is as follows: 3 - the skin does not turn red and is smooth; 2 - part of the skin turns red, loses its luster, and is dry; 1 - most of the skin turns red, does not have lines, and shrinks; 0 - graft rejection makes 80% of the skin necrotic.

**Table 17. Administration regimens for the mouse skin graft rejection model**

| | Group | Drug | N | Dose | Route of administration |
|---|---|---|---|---|---|
| Donor: Balb/c | 1 | Control group (allograft) | 2 | IgG1 10 mg/kg | ip |
| | 2 | Model group (xenograft) | 7 | IgG1 10 mg/kg | ip |
| | 3 | CFZ533 | 7 | 3 mg/kg | ip |
| | 4 | CFZ533 | 7 | 10 mg/kg | ip |
| Acceptor: hCD40 transgenic mouse C57BL6/1 | 5 | 9E6-L4H2 | 7 | 3 mg/kg | ip |
| | 6 | 9E6-L4H2 | 7 | 10 mg/kg | ip |
| | 7 | 2F12-L4H2 | 7 | 3 mg/kg | ip |
| | 8 | 2F12-L4H2 | 7 | 10 mg/kg | ip |
| | 9 | Tacrolimus | 6 | 3 mg/kg | ip |
| | 10 | 9E6-L4H2 + tacrolimus | 6 | 10 mg/kg + 3 mg/kg | ip |
| | 11 | 2F12-L4H2 + tacrolimus | 6 | 10 mg/kg + 3 mg/kg | ip |

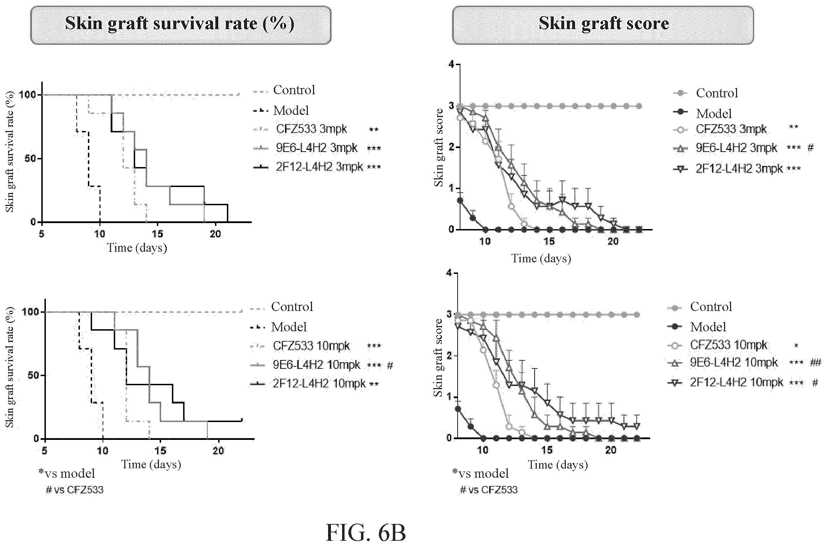

According to the results shown in FIG. 6B and Tables 18-20, both the CD40 antagonistic antibodies significantly improved mouse skin graft scores and prolonged skin graft survival time compared to the model group. Since it could not be determined before if there was rejection, skin graft scoring started from day 8. 9E6-L4H2 and 2F12-L4H2 showed better anti-graft rejection activity than the control antibody CFZ533.

**Table 18. Day-15 skin graft survival rates (%) for anti-CD40 antibodies**

| | Skin graft survival rate (%) | | Skin graft survival rate (%) |
|---|---|---|---|
| Control | 100% | Control | 100% |
| Model | 0% | Model | 0% |
| CFZ533(3mpk) | 0% | CFZ533(10mpk) | 0% |
| 9E6-L4H2(3mpk) | 28.5% | 9E6-L.4112(10mpk) | 14.3% |
| 2F12-L4H2(3mpk) | 28.5% | 2F12-L4H2(10mpk) | 42.9% |

**Table 19. Skin graft scores for anti-CD40 antibodies (3 mpk)**

| | 8 days | 10 days | 15 days | 20 days |
|---|---|---|---|---|
| Control | 3 | 3 | 3 | 3 |
| Model | 0.7 | 0 | 0 | 0 |
| CFZ533(3mpk) | 2.7 | 2.1 | 0 | 0 |
| 9E6-L4H2(3mpk) | 3 | 2.7 | 0.6 | 0 |
| 2F12-L4H2(3mpk) | 2.9 | 2.4 | 0.6 | 0.1 |

**Table 20. Skin graft scores for anti-CD40 antibodies (10 mpk)**

| | 8 days | 10 days | 15 days | 20 days |
|---|---|---|---|---|
| Control | 3 | 3 | 3 | 3 |
| Model | 0.7 | 0 | 0 | 0 |
| CFZ533(10mpk) | 2.9 | 2.1 | 0 | 0 |
| 9E6-L4H2(10mpk) | 3 | 2.7 | 0.3 | 0 |
| 2F12-L4H2(10mpk) | 2.7 | 2.4 | 0.9 | 0.4 |

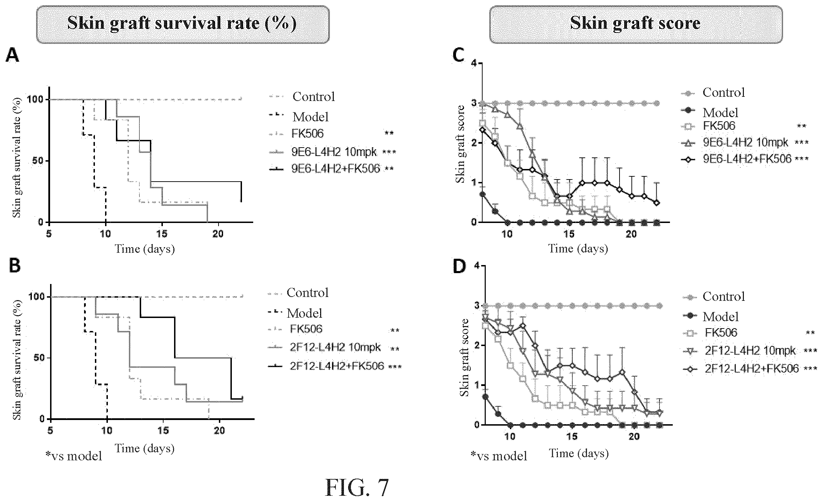

In addition, as shown in FIG. 7 and Tables 21-23, 9E6-L4H2 and 2F12-L4H2 further enhanced the resistance to mouse skin graft rejection after use in combination with tacrolimus.

**Table 21. Day-15 skin graft survival rates for anti-CD40 antibodies in combination with tacrolimus**

| | Skin graft survival rate (%) | | Skin graft survival rate (%) |
|---|---|---|---|
| Control | 100% | Control | 100% |
| Model | 0% | Model | 0% |
| FK506(3mpk) | 16.7% | FK506(3mpk) | 16.7% |
| 9E6-L4H2(10mpk) | 14.3% | 2F12-L4H2(10mpk) | 42.9% |
| 9E6-L4H2(10mpk) +FK506 | 33.3% | 2F12-L4H2(10mpk) +FK506 | 83.3% |

**Table 22. Skin graft scores for anti-CD40 antibodies in combination with tacrolimus**

| | 8 days | 10 days | 15 days | 20 days |
|---|---|---|---|---|
| Control | 3 | 3 | 3 | 3 |
| Model | 0.7 | 0 | 0 | 0 |
| FK506(3mpk) | 2.5 | 1.5 | 0.5 | 0 |
| 9E6-L4H2(10mpk) | 3 | 2.7 | 0.3 | 0 |
| 9E6-L4H2(10mpk)+FK506(3mpk) | 2.3 | 1.5 | 0.7 | 0.7 |

**Table 23. Skin graft scores for anti-CD40 antibodies in combination with tacrolimus**

| | 8 days | 10 days | 15 days | 20 days |
|---|---|---|---|---|
| Control | 3 | 3 | 3 | 3 |
| Model | 0.7 | 0 | 0 | 0 |
| FK506(3mpk) | 2.5 | 1.5 | 0.5 | 0 |
| 2F12-L4H2(10mpk) | 2.7 | 2.4 | 0.9 | 0.4 |
| 2f12-L4H2(10mpk)+FK506(3mpk) | 2.7 | 2.3 | 1.5 | 0.8 |

### Example 14. PK Assays of Humanized Anti-CD40 Antibodies in Human CD40 Transgenic Mice

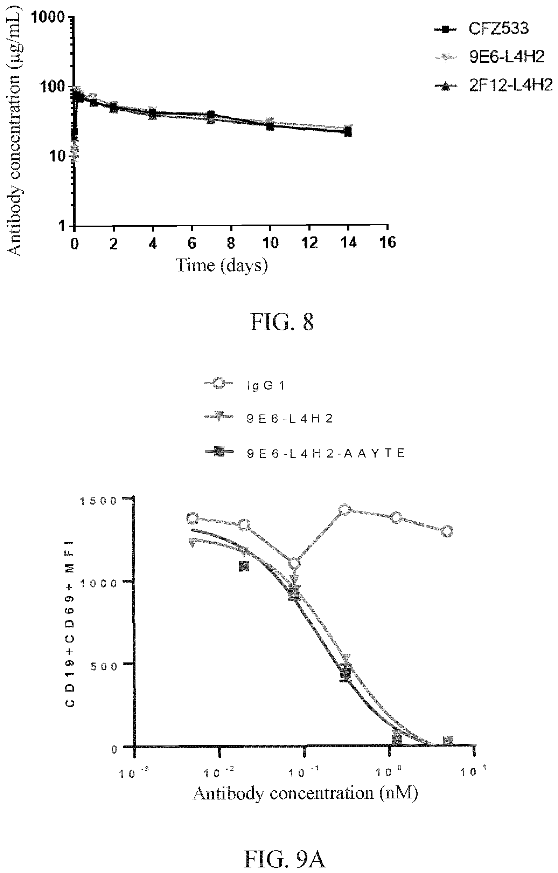

Female human CD40 transgenic mice, 6-7 weeks old, were purchased from Biocytogen Jiangsu Co., Ltd. Housing environment: SPF; production license: SCXK(Jiangsu)-2016-0004; human CD40 transgenic mouse certification number: 320726200100154632. After arrival, the animals were acclimatized for 7 days and randomly grouped. On day 0 of the experiment, each group of mice was intraperitoneally injected with anti-CD40 antibody at 10 mg/kg. Blood samples of 100-150 µL were collected 15 min, 4 h and 8 h after administration, and on days 1, 2, 4, 7, 10 and 14 after administration, anticoagulated with 10 µL of EDTA-K2 (0.1 M), and stored on ice. Mouse plasma was assayed by ELISA for antibody concentration at different time points. The assay is specifically as follows: A goat anti-human IgG Fc antibody (Rockland, Cat. # 609-101-017) was diluted to 2.5 µg/mL with PBS and added to a 96-well plate at 50 µL/well, and the plate was incubated overnight at 4 °C. After three washes with wash buffer, 50 µL of blocking solution was added to each well, and the plate was incubated at 37 °C for 1 h. The mouse plasma and the standard curve of the test antibody were added, and the plate was incubated at 37 °C for 2 h. After three washes with wash buffer, anti-hlgG Fab-HRP (Sigma, Cat. # A0293, 1:10,000) was added at 50 µL/well, and the plate was incubated at room temperature for 1 h. The plate was washed three times with wash buffer. TMB was added at 100 µL/well, and the mixtures were left to react in the dark for 5 min. 0.16 M sulfuric acid was added at 100 µL/well. The OD value at 450 nm was measured with an Envision microplate reader, and the concentrations of CD40 antagonistic antibodies were calculated. According to the results shown in Table 24 and FIG. 8, 9E6-L4H2, 2F12-L4H2 and the control molecule CFZ533 have similar PK characteristics in human CD40 transgenic mice.

**Table 24. PK data for CD40 antagonistic antibodies in hCD40 transgenic mice**

| | CFZ533 | 9E6-L4H2 | 2F12-L4H2 |
|---|---|---|---|
| T1/2 (h) | 214.8 | 226.8 | 257.1 |
| Cmax (mg/L) | 75.95 | 85.8 | 88.78 |
| AUC_{(0-d14)}((h)*(mg/L)) | 12655 | 13439 | 12014 |

### Example 15. Assays for Affinities of Humanized Anti-CD40 Antibodies for Human FcRn

The test antibody was affinity-captured on an anti-human Fab chip, and a series of concentrations of human FcRn antigen (purchased from AcroBiosystem) were allowed to flow over the chip surface. Reaction signals where the reactions were in a steady state were detected in real time using a Biacore instrument. The buffer used in the experiment was HBS-EP + 10× buffer solution (Cat. # BR-1006-69, GE) diluted to 1 × (pH 7.4) with D. I. Water. Fitting was performed on the data obtained from the experiment using a steady state binding model to obtain affinity values. See Table 25. The anti-CD40 antibodies carrying the AAYTE mutation have a higher binding affinity for human FcRn than their parent antibodies.

**Table 25. Assays for the affinities of humanized anti-CD40 antibodies for human FcRn**

| Mobile phase | Stationary phase | KD (M) |
|---|---|---|
| Human FcRn | 2F12-L4H2 | 2.79E-07 |
| | 2F12-L4H2-AAYTE | 4.42E-08 |
| | 9E6-L4H2 | 2.93E-07 |
| | 9E6-L4H2-AAYTE | 3.98E-08 |

### Example 16. Inhibitory Activity of Anti-CD40 Antibodies Carrying AAYTE Mutation in Fc in B Cell Activation Assay System

With reference to the method in Example 10, the inhibitory activity of the anti-CD40 antibodies 2F12-L4H2-AAYTE and 9E6-L4H2-AAYTE, which carry the AAYTE mutation in the Fc, in the B cell activation assay system was measured. According to the results shown in FIGs. 9A to 9B, the Fc-mutation anti-CD40 antibodies have similar B cell inhibition activity to their parent anti-CD40 antibodies.

### Example 17. Inhibitory Activity of Anti-CD40 Antibodies Carrying AAYTE Mutation in Fc in DC Cell Activation Assay System

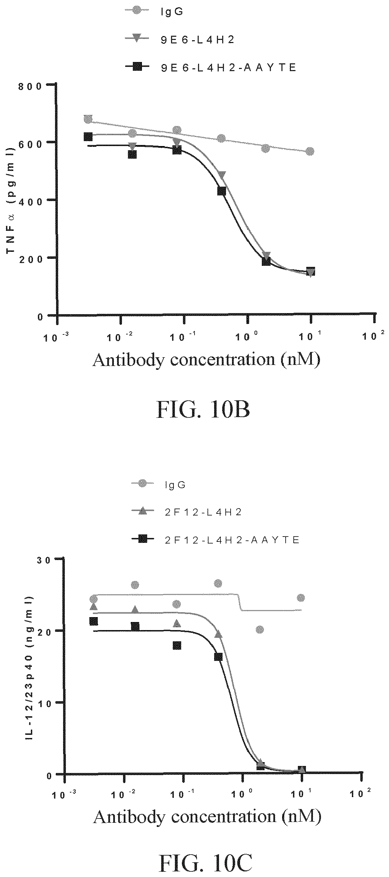

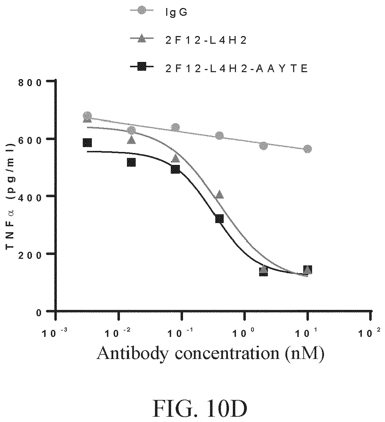

With reference to the method in Example 11, the inhibitory activity of the anti-CD40 antibodies 2F12-L4H2-AAYTE and 9E6-L4H2-AAYTE, which carry the AAYTE mutation in the Fc, in the DC cell activation assay system was measured. According to the results shown in FIGs. 10A to 10D, the Fc-mutation anti-CD40 antibodies have similar DC cell inhibition activity to their parent anti-CD40 antibodies.

## Claims

1. An anti-CD40 antibody or an antigen-binding fragment thereof, comprising a heavy chain variable region (VH) and a light chain variable region (VL), wherein:
a-1) the VH comprises a HCDR1, a HCDR2 and a HCDR3 in a VH set forth in any one of SEQ ID NOs: 67 and 68, and the VL comprises a LCDR1, a LCDR2 and a LCDR3 in a VL set forth in any one of SEQ ID NOs: 63-66;
a-2) the VH comprises a HCDR1, a HCDR2 and a HCDR3 in a VH set forth in SEQ ID NO: 1, and the VL comprises a LCDR1, a LCDR2 and a LCDR3 in a VL set forth in SEQ ID NO: 2;
a-3) the VH comprises a HCDR1, a HCDR2 and a HCDR3 in a VH set forth in SEQ ID NO: 3, and the VL comprises a LCDR1, a LCDR2 and a LCDR3 in a VL set forth in SEQ ID NO: 4;
a-4) the VH comprises a HCDR1, a HCDR2 and a HCDR3 in a VH set forth in SEQ ID NO: 5, and the VL comprises a LCDR1, a LCDR2 and a LCDR3 in a VL set forth in SEQ ID NO: 6;
b-1) the VH comprises a HCDR1, a HCDR2 and a HCDR3 in a VH set forth in any one of SEQ ID NOs: 81 and 82, and the VL comprises a LCDR1, a LCDR2 and a LCDR3 in a VL set forth in any one of SEQ ID NOs: 77-80;
b-2) the VH comprises a HCDR1, a HCDR2 and a HCDR3 in a VH set forth in SEQ ID NO: 7, and the VL comprises a LCDR1, a LCDR2 and a LCDR3 in a VL set forth in SEQ ID NO: 8;
b-3) the VH comprises a HCDR1, a HCDR2 and a HCDR3 in a VH set forth in SEQ ID NO: 9, and the VL comprises a LCDR1, a LCDR2 and a LCDR3 in a VL set forth in SEQ ID NO: 10;
b-4) the VH comprises a HCDR1, a HCDR2 and a HCDR3 in a VH set forth in SEQ ID NO: 11, and the VL comprises a LCDR1, a LCDR2 and a LCDR3 in a VL set forth in SEQ ID NO: 12;
b-5) the VH comprises a HCDR1, a HCDR2 and a HCDR3 in a VH set forth in SEQ ID NO: 13, and the VL comprises a LCDR1, a LCDR2 and a LCDR3 in a VL set forth in SEQ ID NO: 14;
b-6) the VH comprises a HCDR1, a HCDR2 and a HCDR3 in a VH set forth in SEQ ID NO: 15, and the VL comprises a LCDR1, a LCDR2 and a LCDR3 in a VL set forth in SEQ ID NO: 16;
b-7) the VH comprises a HCDR1, a HCDR2 and a HCDR3 in a VH set forth in SEQ ID NO: 17, and the VL comprises a LCDR1, a LCDR2 and a LCDR3 in a VL set forth in SEQ ID NO: 18;
c) the VH comprises a HCDR1, a HCDR2 and a HCDR3 in a VH set forth in SEQ ID NO: 19, and the VL comprises a LCDR1, a LCDR2 and a LCDR3 in a VL set forth in SEQ ID NO: 20; or
d) the VH comprises a HCDR1, a HCDR2 and a HCDR3 in a VH set forth in SEQ ID NO: 21, and the VL comprises a LCDR1, a LCDR2 and a LCDR3 in a VL set forth in SEQ ID NO: 22;
wherein the CDRs are defined according to the Kabat, IMGT, Chothia, AbM or Contact numbering scheme, preferably the Kabat numbering scheme.

2. The anti-CD40 antibody or the antigen-binding fragment thereof according to claim 1, comprising:
1) a heavy chain HCDR1, a heavy chain HCDR2 and a heavy chain HCDR3, comprising the sequences set forth in SEQ ID NOs: 23, 24 and 25, respectively; and a light chain LCDR1, a light chain LCDR2 and a light chain LCDR3, comprising the sequences set forth in SEQ ID NOs: 74, 75 and 76, respectively;
2) a heavy chain HCDR1, a heavy chain HCDR2 and a heavy chain HCDR3, comprising the sequences set forth in SEQ ID NOs: 88, 89 and 90, respectively; and a light chain LCDR1, a light chain LCDR2 and a light chain LCDR3, comprising the sequences set forth in SEQ ID NOs: 91, 37 and 92, respectively;
3) a heavy chain HCDR1, a heavy chain HCDR2 and a heavy chain HCDR3, comprising the sequences set forth in SEQ ID NOs: 51, 52 and 53, respectively; and a light chain LCDR1, a light chain LCDR2 and a light chain LCDR3, comprising the sequences set forth in SEQ ID NOs: 54, 55 and 56, respectively; or
4) a heavy chain HCDR1, a heavy chain HCDR2 and a heavy chain HCDR3, comprising the sequences set forth in SEQ ID NOs: 57, 58 and 59, respectively; and a light chain LCDR1, a light chain LCDR2 and a light chain LCDR3, comprising the sequences set forth in SEQ ID NOs: 60, 61 and 62, respectively;
wherein preferably, the anti-CD40 antibody or the antigen-binding fragment thereof in 1) comprises:
1-1) a heavy chain HCDR1, a heavy chain HCDR2 and a heavy chain HCDR3, comprising the sequences set forth in SEQ ID NOs: 23, 24 and 25, respectively; and a light chain LCDR1, a light chain LCDR2 and a light chain LCDR3, comprising the sequences set forth in SEQ ID NOs: 29, 30 and 73, respectively;
1-2) a heavy chain HCDR1, a heavy chain HCDR2 and a heavy chain HCDR3, comprising the sequences set forth in SEQ ID NOs: 23, 24 and 25, respectively; and a light chain LCDR1, a light chain LCDR2 and a light chain LCDR3, comprising the sequences set forth in SEQ ID NOs: 26, 27 and 28, respectively; or
1-3) a heavy chain HCDR1, a heavy chain HCDR2 and a heavy chain HCDR3, comprising the sequences set forth in SEQ ID NOs: 23, 24 and 25, respectively; and a light chain LCDR1, a light chain LCDR2 and a light chain LCDR3, comprising the sequences set forth in SEQ ID NOs: 29, 27 and 32, respectively;
preferably, the anti-CD40 antibody or the antigen-binding fragment thereof in 2) comprises:
2-1) a heavy chain HCDR1, a heavy chain HCDR2 and a heavy chain HCDR3, comprising the sequences set forth in SEQ ID NOs: 39, 47 and 49, respectively; and a light chain LCDR1, a light chain LCDR2 and a light chain LCDR3, comprising the sequences set forth in SEQ ID NOs: 87, 50 and 48, respectively;
2-2) a heavy chain HCDR1, a heavy chain HCDR2 and a heavy chain HCDR3, comprising the sequences set forth in SEQ ID NOs: 33, 34 and 35, respectively; and a light chain LCDR1, a light chain LCDR2 and a light chain LCDR3, comprising the sequences set forth in SEQ ID NOs: 36, 37 and 38, respectively;
2-3) a heavy chain HCDR1, a heavy chain HCDR2 and a heavy chain HCDR3, comprising the sequences set forth in SEQ ID NOs: 39, 40 and 41, respectively; and a light chain LCDR1, a light chain LCDR2 and a light chain LCDR3, comprising the sequences set forth in SEQ ID NOs: 42, 37 and 43, respectively;
2-4) a heavy chain HCDR1, a heavy chain HCDR2 and a heavy chain HCDR3, comprising the sequences set forth in SEQ ID NOs: 39, 44 and 35, respectively; and a light chain LCDR1, a light chain LCDR2 and a light chain LCDR3, comprising the sequences set forth in SEQ ID NOs: 45, 37 and 46, respectively; or
2-5) a heavy chain HCDR1, a heavy chain HCDR2 and a heavy chain HCDR3, comprising the sequences set forth in SEQ ID NOs: 39, 47 and 41, respectively; and a light chain LCDR1, a light chain LCDR2 and a light chain LCDR3, comprising the sequences set forth in SEQ ID NOs: 36, 37 and 48, respectively;
more preferably, the anti-CD40 antibody or the antigen-binding fragment thereof in 1-1) comprises:
a heavy chain HCDR1, a heavy chain HCDR2 and a heavy chain HCDR3, comprising the sequences set forth in SEQ ID NOs: 23, 24 and 25, respectively; a light chain LCDR1, comprising the sequence set forth in SEQ ID NO: 29; a light chain LCDR2, comprising the sequence set forth in SEQ ID NO: 30; and a light chain LCDR3, comprising the sequence set forth in any one of SEQ ID NOs: 69-72;
more preferably, the anti-CD40 antibody or the antigen-binding fragment thereof in 2-1) comprises:
a heavy chain HCDR1, a heavy chain HCDR2 and a heavy chain HCDR3, comprising the sequences set forth in SEQ ID NOs: 39, 47 and 49, respectively; a light chain LCDR1, comprising the sequence set forth in any one of SEQ ID NOs: 83-86; a light chain LCDR2, comprising the sequence set forth in SEQ ID NO: 50; and a light chain LCDR3, comprising the sequence set forth in SEQ ID NO: 48.

3. The anti-CD40 antibody or the antigen-binding fragment thereof according to claim 1 or 2, being a recombinant antibody, a rabbit antibody, a chimeric antibody, a humanized antibody, a fully human antibody or an antigen-binding fragment thereof.

4. The anti-CD40 antibody or the antigen-binding fragment thereof according to claim 3, wherein:
of the humanized antibody or the antigen-binding fragment thereof, heavy chain framework regions are derived from IGHV2-26*01, IGHV4-30-4*02, IGHV4-4*08 and IGHJ1*01, and/or light chain framework regions are derived from IGkV1-13*02, IGkV1-9*01, IGkV1-6*01 and IGKJ4*01;
preferably, heavy chain framework regions FR1-FR3 are derived from IGHV2-26*01, IGHV4-30-4*02 or IGHV4-4*08, and a heavy chain framework region FR4 is derived from IGHJ1*01; light chain framework regions FR1-FR3 are derived from IGkV1-13 * 02, IGkV1-9*01 or IGkV1-6*01, and a light chain framework region FR4 is derived from IGKJ4*01.

5. The anti-CD40 antibody or the antigen-binding fragment thereof according to any one of claims 1-4, wherein:
A-1) the amino acid sequence of the VH is set forth in SEQ ID NO: 67 or has at least 90% identity thereto, and the amino acid sequence of the VL is set forth in any one of SEQ ID NOs: 63-66 or has at least 90% identity thereto;
A-2) the amino acid sequence of the VH is set forth in SEQ ID NO: 68 or has at least 90% identity thereto, and the amino acid sequence of the VL is set forth in any one of SEQ ID NOs: 63-66 or has at least 90% identity thereto;
A-3) the amino acid sequence of the VH is set forth in SEQ ID NO: 1 or has at least 90% identity thereto, and the amino acid sequence of the VL is set forth in SEQ ID NO: 2 or has at least 90% identity thereto;
A-4) the amino acid sequence of the VH is set forth in SEQ ID NO: 3 or has at least 90% identity thereto, and the amino acid sequence of the VL is set forth in SEQ ID NO: 4 or has at least 90% identity thereto;
A-5) the amino acid sequence of the VH is set forth in SEQ ID NO: 5 or has at least 90% identity thereto, and the amino acid sequence of the VL is set forth in SEQ ID NO: 6 or has at least 90% identity thereto;
B-1) the amino acid sequence of the VH is set forth in SEQ ID NO: 81 or has at least 90% identity thereto, and the amino acid sequence of the VL is set forth in any one of SEQ ID NOs: 77-80 or has at least 90% identity thereto;
B-2) the amino acid sequence of the VH is set forth in SEQ ID NO: 82 or has at least 90% identity thereto, and the amino acid sequence of the VL is set forth in any one of SEQ ID NOs: 77-80 or has at least 90% identity thereto;
B-3) the amino acid sequence of the VH is set forth in SEQ ID NO: 7 or has at least 90% identity thereto, and the amino acid sequence of the VL is set forth in SEQ ID NO: 8 or has at least 90% identity thereto;
B-4) the amino acid sequence of the VH is set forth in SEQ ID NO: 9 or has at least 90% identity thereto, and the amino acid sequence of the VL is set forth in SEQ ID NO: 10 or has at least 90% identity thereto;
B-5) the amino acid sequence of the VH is set forth in SEQ ID NO: 11 or has at least 90% identity thereto, and the amino acid sequence of the VL is set forth in SEQ ID NO: 12 or has at least 90% identity thereto;
B-6) the amino acid sequence of the VH is set forth in SEQ ID NO: 13 or has at least 90% identity thereto, and the amino acid sequence of the VL is set forth in SEQ ID NO: 14 or has at least 90% identity thereto;
B-7) the amino acid sequence of the VH is set forth in SEQ ID NO: 15 or has at least 90% identity thereto, and the amino acid sequence of the VL is set forth in SEQ ID NO: 16 or has at least 90% identity thereto;
B-8) the amino acid sequence of the VH is set forth in SEQ ID NO: 17 or has at least 90% identity thereto, and the amino acid sequence of the VL is set forth in SEQ ID NO: 18 or has at least 90% identity thereto;
C) the amino acid sequence of the VH is set forth in SEQ ID NO: 19 or has at least 90% identity thereto, and the amino acid sequence of the VL is set forth in SEQ ID NO: 20 or has at least 90% identity thereto; or
D) the amino acid sequence of the VH is set forth in SEQ ID NO: 21 or has at least 90% identity thereto, and the amino acid sequence of the VL is set forth in SEQ ID NO: 22 or has at least 90% identity thereto.

6. The anti-CD40 antibody or the antigen-binding fragment thereof according to any one of the preceding claims, further comprising an Fc region of an IgG antibody, wherein preferably, the IgG antibody is an IgG1, IgG2 or IgG4 antibody; more preferably, the Fc region is an Fc region of IgG1 containing the mutation N297A, or an Fc region of IgG1 having any one or more of the mutations L234A, L235A, M252Y, S254T and T256E.

7. The anti-CD40 antibody or the antigen-binding fragment thereof according to any one of the preceding claims, wherein the antigen-binding fragment is an scFv, Fv, Fab or Fab' fragment.

8. The anti-CD40 antibody or the antigen-binding fragment thereof according to any one of the preceding claims, wherein:
the full-length amino acid sequence of a heavy chain is set forth in SEQ ID NO: 93 or 97 or has at least 90% identity thereto, and the full-length amino acid sequence of a light chain is set forth in SEQ ID NO: 94 or has at least 90% identity thereto; or
the full-length amino acid sequence of a heavy chain is set forth in SEQ ID NO: 95 or 98 or has at least 90% identity thereto, and the full-length amino acid sequence of a light chain is set forth in SEQ ID NO: 96 or has at least 90% identity thereto.

9. The anti-CD40 antibody or the antigen-binding fragment thereof according to any one of claims 1-8, having at least one of the following:
i) binding to human CD40 with a K_{D} of 10 nM or lower; and
ii) having no significant agonistic activity.

10. An isolated polynucleotide, encoding the anti-CD40 antibody or the antigen-binding fragment thereof according to any one of claims 1-9.

11. A vector, comprising the polynucleotide according to claim 10.

12. A host cell, comprising the polynucleotide according to claim 10 or the vector according to claim 11.

13. A CD40-binding molecule, comprising the anti-CD40 antibody or the antigen-binding fragment thereof according to any one of claims 1-9.

14. A pharmaceutical composition, comprising the anti-CD40 antibody or the antigen-binding fragment thereof according to any one of claims 1-9, and at least one pharmaceutically acceptable excipient, diluent or carrier.

15. The pharmaceutical composition according to claim 14, further comprising tacrolimus.

16. A method for treating an autoimmune disease, comprising:
administering to a subject in need thereof a therapeutically effective amount of the anti-CD40 antibody or the antigen-binding fragment thereof according to any one of claims 1-9, or the pharmaceutical composition according to claim 14, wherein
the autoimmune disease is preferably Sjögren's syndrome, multiple sclerosis or lupus erythematosus, and is more preferably systemic lupus erythematosus.

17. Use of the anti-CD40 antibody or the antigen-binding fragment thereof according to any one of claims 1-9 for the preparation of a medicament for treating an autoimmune disease, wherein
the autoimmune disease is preferably Sjögren's syndrome, multiple sclerosis or lupus erythematosus, and is more preferably systemic lupus erythematosus.

18. A method for treating graft-versus-host disease or alleviating graft rejection, comprising:
administering to a subject in need thereof a therapeutically effective amount of the anti-CD40 antibody or the antigen-binding fragment thereof according to any one of claims 1-9, or the pharmaceutical composition according to any one of claims 14-15, wherein the graft is preferably a solid organ graft, and is more preferably a liver, kidney, heart or lung graft.

19. Use of the anti-CD40 antibody or the antigen-binding fragment thereof according to any one of claims 1-9 in combination with tacrolimus for the preparation of a medicament for treating graft-versus-host disease or alleviating graft rejection, wherein
the graft is preferably a solid organ graft, and is more preferably a liver, kidney, heart or lung graft.
